# EUROPEAN PATENT APPLICATION

(11) **EP 3 696 192 A1**
(43) Date of publication of application: **19.08.2020**
(21) Application number: 19382105.5
(22) Date of filing: 15.02.2019
(51) Int. Cl.: C07K 16/18, A61P 25/28

(54) **THERAPEUTIC TARGET AND MONOCLONAL ANTIBODIES AGAINST IT FOR THE DIAGNOSIS AND TREATMENT OF ALZHEIMER´S DISEASE**

(71) Applicant: Consejo Superior De Investigaciones Científicas, 28006 Madrid (ES); Instituto de Salud Carlos III, 28029 Madrid (ES)
(72) Inventor: BOVOLENTA NICOLAO, Paola, 28049 Madrid (ES); ESTEVE PASTOR, Pilar, 28049 Madrid (ES); RUEDA CARRASCO, Javier, 28049 Madrid (ES); MATEO RUIZ, María Inés, 28049 Madrid (ES); MARTIN BERMEJO, María Jesús, 28049 Madrid (ES); DOMINGUEZ RODRIGUEZ, Mercedes, 28029 Madrid (ES); MORENO IRUELA, Inmaculada, 28029 Madrid (ES)
(74) Representative: Pons

(57) **Abstract**

The invention provides a therapeutic target for the screening of drugs for the treatment of Alzheimer's disease (AD), as well as neutralizing monoclonal antibodies against said target and their use for the treatment of AD. The invention also refers to a kit comprising the antibodies and to an *in vitro* method for the diagnosis of AD in which preferably the antibodies or the kit comprising them are used.

SEQUENCE LISTING

<110>  Consejo Superior de Investigaciones Científicas (CSIC)
       Instituto de Salud Carlos III (ISCIII)

<120>  THERAPEUTIC TARGET AND MONOCLONAL ANTIBODIES AGAINST IT FOR THE
       DIAGNOSIS AND TREATMENT OF ALZHEIMER´S DISEASE

<130>  EP1641.1438

<160>  14

<170>  PatentIn version 3.5

<210>  1
<211>  138
<212>  PRT
<213>  Artificial Sequence

<220>
<223>  Light chain of the variable region of the first mAb of the
       invention (mAb 10.5.6)

<400>  1

Asp Ile Gln Met Thr Gln Thr Gln Val Phe Val Tyr Met Leu Leu Trp
1               5                   10                  15

Leu Ser Gly Val Asp Gly Asp Ile Val Met Thr Gln Ser Gln Lys Phe
            20                  25                  30

Met Ser Thr Ser Val Gly Asp Arg Val Ser Val Thr Cys Lys Ala Ser
        35                  40                  45

Gln Asn Val Arg Thr Asn Val Ala Trp Tyr Gln Gln Arg Pro Gly Gln
    50                  55                  60

Ser Pro Lys Ala Leu Ile Tyr Ser Ala Ser Tyr Arg Tyr Ser Gly Val
65                  70                  75                  80

Pro Asp Arg Phe Thr Gly Ser Gly Ser Gly Thr Asp Phe Thr Leu Thr
                85                  90                  95

Ile Ser Asn Val Gln Ser Glu Asp Leu Ala Lys Tyr Phe Cys Gln Gln
            100                 105                 110

Phe Asn Thr Phe Pro Tyr Thr Phe Gly Gly Gly Thr Lys Leu Glu Ile
        115                 120                 125

Lys Arg Ala Asp Ala Ala Pro Thr Val Ser
    130                 135

<210>  2
<211>  120

<212>  PRT
<213>  Artificial Sequence

<220>
<223>  Heavy chain of the variable region of the first mAb of the
       invention (mAb 10.5.6)

<400>  2

Asp Ala Glu Leu Val Lys Pro Gly Ala Ser Val Lys Ile Ser Cys Lys
1               5                   10                  15

Ala Ser Gly Tyr Thr Phe Thr Asp His Ala Ile His Trp Val Lys Gln
            20                  25                  30

Lys Pro Gln Gln Gly Leu Glu Trp Ile Gly Tyr Ile Ser Pro Gly Asn
        35                  40                  45

Gly Asp Ile Lys Tyr Asn Glu Lys Phe Gln Gly Lys Ala Thr Leu Thr
    50                  55                  60

Ala Asp Lys Ser Ser Ser Thr Ala Tyr Met Gln Leu Asn Ser Leu Ile
65                  70                  75                  80

Ser Glu Asp Ser Ala Val Tyr Phe Cys Lys Arg Tyr Gly Asn Tyr Val
                85                  90                  95

Tyr Trp Gly Gln Gly Thr Leu Val Thr Val Ser Ala Ala Lys Thr Thr
            100                 105                 110

Pro Pro Ser Asp Ser Pro Leu Ala
        115                 120


<210>  3
<211>  127
<212>  PRT
<213>  Artificial Sequence

<220>
<223>  Heavy chain of the variable region of the second mAb of the
       invention (mAb 17.08.13)

<400>  3

Met Leu Val Glu Ser Gly Gly Gly Leu Val Gln Pro Gly Gly Phe Leu
1               5                   10                  15

Arg Leu Ser Cys Ala Thr Ser Gly Phe Thr Phe Thr Asp Asn Tyr Met
            20                  25                  30

Asn Trp Val Arg Gln Pro Pro Gly Lys Ala Leu Glu Trp Leu Gly Leu
        35                  40                  45

Ile Arg Asn Lys Ala Asn Gly Tyr Thr Thr Glu Tyr Ser Ala Ser Val
    50                  55                  60

Lys Gly Arg Phe Thr Ile Ser Arg Asp Asn Ser Gln Asn Ile Val Tyr
65                  70                  75                  80

Leu Gln Met Asn Thr Leu Arg Ala Glu Asp Ser Gly Thr Tyr Phe Cys
                85                  90                  95

Ala Arg Ala Gly Tyr Phe Asp Asp Trp Gly Ala Gly Thr Thr Val Thr
            100                 105                 110

Val Ser Ser Ala Lys Thr Thr Pro Pro Ser Asp Tyr His Thr Gly
        115                 120                 125

<210>  4
<211>  415
<212>  DNA
<213>  Artificial Sequence

<220>
<223>  Nucleotide sequence encoding the light chain of the variable
       region of the first mAb of the invention (mAb 10.5.6)

<400>  4
gatatccaga tgacacagac tcaggtcttt gtatacatgt tgctgtggtt gtctggtgtt      60

gatggagaca ttgtgatgac ccagtctcaa aaattcatgt ccacatcagt aggagacagg     120

gtcagcgtca cctgcaaggc cagtcagaat gtgcgtacta atgtagcctg gtatcaacag     180

agaccagggc aatctcctaa agcactgatt tactcggcat cctaccggta cagtggagtc     240

cctgatcgct tcacaggcag tggatctggg acagatttca ctctcaccat cagcaatgtg     300

cagtctgaag acttggcaaa gtatttctgt cagcaattta acacctttcc gtacacgttc     360

ggagggggga ccaagctgga aataaaacgg gctgatgctg caccaactgt atcca           415

<210>  5
<211>  368
<212>  DNA
<213>  Artificial Sequence

<220>
<223>  Nucleotide sequence encoding the heavy chain of the variable
       region of the first mAb of the invention (mAb 10.5.6)

<400>  5
caggtgcagc tgaaggagtc tgacgctgag ttggtgaaac ctggggcttc agtgaagata      60

tcctgcaagg cttctggcta caccttcact gaccatgcta ttcactgggt gaagcagaag     120

cctcaacagg gcctggaatg gattggatat atttctcccg gaaatggtga tattaagtac     180

aatgagaagt tccagggcaa ggccacactg actgcagaca aatcctccag tactgcctac     240

atgcagctca acagcctgat atctgaggat tctgcagtgt atttctgtaa aagatatggt 300

aactacgttt actggggcca agggactctg gtcactgtct ctgcagccaa aacgacaccc 360

ccatctga 368

<210> 6
<211> 369
<212> DNA
<213> Artificial Sequence

<220>
<223> Nucleotide sequence encoding the heavy chain of the variable
      region of the second mAb of the invention (mAb 17.08.13)

<400> 6
gctggtggaa tctggaggag gcttggtaca gcctgggggt tttctgagac tctcctgtgc 60

aacttctggg ttcaccttca ctgataacta catgaactgg gtccgccagc ctccaggaaa 120

ggcacttgag tggttgggtt tgattagaaa caaagctaat ggttacacaa cagagtacag 180

tgcatctgtg aagggtcggt ttaccatctc cagagataat tcccaaaaca tcgtctatct 240

tcaaatgaac accctgagag ctgaggacag tggcacttat ttctgtgcaa gagcagggta 300

cttcgatgac tggggcgcag ggaccacggt caccgtctcc tcagccaaaa cgacaccccc 360

atctgacta 369

<210> 7
<211> 20
<212> DNA
<213> Artificial Sequence

<220>
<223> GAPDH forward primer

<400> 7
accacagtcc atgccatcac 20

<210> 8
<211> 20
<212> DNA
<213> Artificial Sequence

<220>
<223> GAPDH reverse primer

<400> 8
tccaccaccc tgttgctgta 20

<210> 9
<211> 21
<212> DNA
<213> Artificial Sequence

<220>

<223>  Axin2 forward primer

<400>  9
ctccttggag gcaagagctt c                                                21


<210>  10
<211>  21
<212>  DNA
<213>  Artificial Sequence

<220>
<223>  Axin2 reverse primer

<400>  10
gactggccac gcagcaccgc t                                                21


<210>  11
<211>  18
<212>  DNA
<213>  Artificial Sequence

<220>
<223>  APP forward primer

<400>  11
atgtgcagaa tggaaagt                                                    18


<210>  12
<211>  18
<212>  DNA
<213>  Artificial Sequence

<220>
<223>  APP reverse primer

<400>  12
cagcatacaa actctacc                                                    18


<210>  13
<211>  23
<212>  DNA
<213>  Artificial Sequence

<220>
<223>  Adam10 forward primer

<400>  13
gctgtgattg ctcagatatc cag                                              23


<210>  14
<211>  20
<212>  DNA
<213>  Artificial Sequence

<220>
<223>  Adam10 reverse primer

<400>  14

tctttgcact ggtcactgta 20

## Description

This invention belongs to the field of methods for the diagnosis and treatment of Alzheimer's disease (AD). Particularly, the invention refers to therapeutic monoclonal antibodies capable of binding and neutralizing the activity of a specific molecular target (SFRP1) that contributes to the generation of Aβ aggregates that accumulate in amyloid plaques (APs) in the brain of AD patients. The invention, therefore, refers to these antibodies, kits and pharmaceutical compositions comprising them and their use for the treatment and/or diagnosis of AD in a subject.

### BACKGROUND ART

The deposition of aggregated Aβ peptides -derived from the pro-amyloidogenic processing of the Amyloid Precursor Protein (APP)- into characteristic plaques is a distinctive feature of Alzheimer's disease. Aβ formation can be prevented by alternative APP processing via the metalloprotease ADAM10. ADAM10 is a constitutive α-secretase of the brain that cleaves APP blocking the generation of Aβ peptides and releasing a soluble extracellular fragment, known as sAPPα. Mutations in the ADAM10 pro-domain, which lower its α-secretase activity and shift APP processing towards the pro-amyloidogenic pathway, co-segregate with some sporadic Alzheimer's disease cases. This raises the possibility that a poor ADAM10 activity might be among the common triggers of amyloid deposition, contributing to Alzheimer's disease pathogenesis.

A human monoclonal anti-beta-amyloid antibody, named Aducanumab, has been proved to reduce Aβ oligomers in Alzheimer's disease (Sevigny, J., et al., 2016, Nature 537 (7618), 50-56; WO2017211827). This antibody targets soluble oligomeric forms of Aβ peptides with the aim of reducing its buildup. Other disclosed anti-amyloid beta antibodies are Bapineuzumab, Solanezumab, Gantenerumab, Crenezumab, BAN2401 and Ponezumab. These antibody-based therapies for treating Alzheimer's disease are primarily aimed at targeting unwanted accumulation of Aβ peptides. As a result, unwanted Aβ peptides are cleared from the brain. These treatments, however, result only in partial success (Van Dyck, 2018; Biol. Psychiatry, 83, 311-319). Thus, unfortunately, despite the efforts made up to date for developing therapeutic treatments based on the reduction of Aβ plaques, the disease is not arrested (Golde, T.E., et al., 2011, Neuron 69, 203-213; Fowler, S.W., et al., 2014, J Neurosci 34, 7871-7885; and Sevigny, J., et al., 2016, Nature 537, 50-56; Van Dyck, 2018; Biol. Psychiatry, 83, 311-319), perhaps because the factors that trigger amyloid deposition are still unknown or treatments were attempted when the disease was too advanced. Successful treatments for Alzheimer's disease need to account for the genetic complexity of the disease.

Secreted frizzled-related protein 1 (SFRP1) is a member of the SFRP family that contains a cysteine-rich domain homologous to the putative Wnt-binding site of Frizzled proteins. SFRPs act as soluble modulators of Wnt signalling; so that SFRP1 has been studied for its role in several human tumor entities, since it counteracts Wnt-induced effects at high concentrations and promotes them at lower concentrations (Bovolenta, P., et al., 2008, Journal of cell science, 121, 737-746). The role of SFRP1 as a tumor suppressor has been proposed in many cancers, based on its loss in patient tumors. Its frequent inactivation by methylation-induced silencing is consistent with it behaving as a tumor suppressor. Also, the SFRP1 gene is located in a region on chromosome 8 that is frequently lost in many cancer types. Loss of SFRP1 protein expression is associated with poor overall survival (OS) in patients with early breast cancer (pT1 tumours).

Identifying novel players involved in Alzheimer's disease pathogenesis is still crucial in the field of neuromedicine, since said molecules could be promising therapeutic targets for developing alternative and improved treatments against Alzheimer' s disease as well as for designing alternative and improved diagnostic methods for the disease.

### DESCRIPTION OF THE INVENTION

In this invention, the Secreted Frizzled Related Protein 1 (SFRP1) has been identified as a therapeutic target for neurodegeneration, preferably for Alzheimer's disease (AD), treatment. Specific and neutralizing monoclonal antibodies against it are also provided herein for the treatment and diagnosis of these diseases.

Therefore, in the present invention, a new therapeutic target for the development or screening of drugs for the treatment of AD has been identified. Specifically, it has been demonstrated by the inventors that the SFRP1 protein, as well as its mRNA, is significantly increased in the brain and cerebrospinal fluid (CSF) of AD patients. In the brain, SFRP1 strongly binds Aβ and forms aggregates that accumulate in amyloid plaques (APs). Thus, down-modulation of ADAM10 by its secreted endogenous inhibitor SFRP1 is a common AD trait. It is, therefore, proposed herein the SFRP1 protein, SFRP1 mRNA or *Sfrp1* gene as a pharmacological target for the treatment and/or prevention of AD.

*Sfrp1* overexpression in an AD-like mouse model anticipates the appearance of APs and dystrophic neurites, whereas its genetic inactivation or the infusion of an anti-SFRP1 (α-SFRP1) neutralizing antibody, shifts APP processing towards the non-amyloidogenic pathway. Therefore, in the present invention it has been evidenced that decreased SFRP1 levels lower AP accumulation, improves AD-related histopathological traits and prevents long-term potentiation (LTP) loss and cognitive deficits' appearance. This invention unveils therefore SFRP1 as a crucial novel player of AD pathogenesis and promising new target for AD treatment.

Examples shown in this application evidence that knocking out SFRP1 largely prevents the appearance of pathogenic characteristics in AD, strongly delaying the progressive dysfunctions associated to this disease. SFRP1 inactivation protects from the appearance of behavioural deficits. Anti-SFRP1 treatment through a neutralizing mAb antibody prevented the formation and enlargement of toxic plaques in brain. Manipulations of SFRP1 levels showed that the absence or neutralization of SFRP1 activity suffices to prevent cognitive loss and restore the synaptic function in amyloidosis mice models. The accumulation of SFRP1 in APs and its significant upregulation in brain parenchyma and CSF of AD patients makes also a strong case in favor of SFRP1 implication in disease pathogenesis. In mice, SFRP1 neutralization results in a decrease of pathogenic signs, proving that lowering SFRP1 levels has a positive therapeutic effect. Thus, targeting SFRP1 represents an AD therapeutic avenue.

Accordingly, the present invention further provides neutralizing monoclonal antibodies (mAbs) that specifically bind the SFRP1 protein and inhibit its activity. Since levels of this protein are elevated in AD pathogenesis, these antibodies are useful not only for the treatment and/or prevention of the disease but also for the specific diagnosis of the same. These mAbs of the invention are capable of detecting very low amounts of the SFRP1 protein in a biological sample therefore they can be used in a sensitive and reliable diagnosis method of AD.

Thus, and based on the correlation between SFRP1 upregulation and AD pathological state, the present invention also provides a specific and sensitive AD diagnosis method, preferably an ELISA assay, in which the newly generated α-SFRP1 mAbs mentioned in the paragraph above are used. Examples of this application show that SFRP1 protein is elevated in both the soluble and RIPA-insoluble fractions of entorhinal and frontal cortex extracts from AD patients at pre-symptomatic, mild and advanced neuropathological stages of the disease (Braak and Braak (BB) BB I-II/0-A; III-IV/0-C; BB V-VI/B-C), when compared to age-matched control samples. Thus, the diagnosis method proposed herein presents the advantage that it can be carried out for detecting the disease from early (pre-symptomatic) stages.

Furthermore, since SFRP1 is involved in other pathologies, the antibodies provided in this invention may also be used for the sensitive and reliable diagnosis of other diseases. In particular, since this protein has been identified as a tumor suppressor (Matsuda et al., Breast Cancer Res. 2009; 11, R32; Bernemann et al. Molecular Cancer., 2014,13, 174), the antibodies described herein are also useful for the diagnosis or prognosis of cancer.

### Drug screening method and pharmaceutical composition comprising inhibitors of SFRP1 for the treatment and/or prevention of AD

One aspect of the invention refers to the use of the *Sfrp1* gene (nucleotide sequence encoding SFRP1), the SFRP1 mRNA or the SFRP1 protein (SFRP1 amino acid sequence) as a pharmacological (therapeutic) target for designing and/or screening molecules, compounds, compositions, drugs or the like, useful for the treatment and/or prevention of AD.

The term "Alzheimer's Disease" or "AD" relates to a chronic neurodegenerative disease that leads to cognitive impairment and/or behavioral disorders. It is characterised in its typical form by a progressive loss of memory and other mental capabilities as the nerve cells degenerate and/or die and various areas of the brain become atrophied. Alzheimer's disease includes familial Alzheimer's disease (FAD) or sporadic Alzheimer's disease (SAD).

AD neuropathology is characterized by accumulation of Aβ peptides and neurofibrillary tangles comprising Tau in the Central Nervous System, synaptic loss, and neuronal death. Specifically, accumulation of Aβ peptides in the form of amyloid plaques or soluble Aβ oligomers is involved in AD progression.

The term "pharmacological or therapeutic target", as used in the present invention, relates to the SFRP1 protein, the *Sfrp1* gene or the SFRP1 mRNA, which are useful for studying the biochemical effect of molecules capable of binding and neutralizing the activity of the same. The molecules, compounds, compositions, drugs or the like, of interest are those that exercise a blocking or neutralizing effect that prevents the activity of the SFRP1 protein, the *Sfrp1* gene or the SFRP1 mRNA. These molecules may be, for example, antibodies, antigen binding fragments of antibodies, aptamers, interference RNAs (iRNAs), small interfering RNAs (siRNAs), a short hairpin RNA (shRNA), a microRNA (miRNA), an antisense oligonucleotide, a peptide, a peptidomimetic, a small molecule, a chemical agent or the like.

Another aspect of the invention refers to an *in vitro* method of screening for a therapeutic agent useful for treating and/or preventing AD or a condition in which an accumulation of toxic Aβ peptides exists, comprising:
(a) contacting the SFRP1 protein, the SFRP1 mRNA or the *Sfrp1* gene, preferably a cell (more preferably an isolated cell) that expresses the SFRP1 protein, even more preferably that overexpresses the SFRP1 protein, with a therapeutic agent to be tested;
(b) detecting the inhibition or blockage of the activity of said protein, or the decrease in the expression levels of its mRNA or gene, by the therapeutic agent; and
(c) identifying the therapeutic agent as useful for treating and/or preventing AD if said agent inhibits or blocks the activity of the protein or decreases the expression levels of its mRNA or gene.

The term "activity" as used herein refers to protein biological or chemical function. The presence of activity of the SFRP1 protein is detected, for instance, when ADAM10 activity is attenuated and an accumulation of toxic Aβ peptides, preferably in APs, is observed. In a preferred embodiment, the activity of the SFRP1 protein, determined preferably as the accumulation of toxic Aβ peptides, is measured or detected by a method selected from Western blot, electrophoresis gels, immunoprecipitation, protein arrays, enzyme-linked immunosorbent assays (ELISA), chromatography, a fluorescence assay, immunohistochemistry, a luciferase assay, an enzymatic assay, by means of MRI or any other diagnostic imaging technique, or, for example, using chromatographic techniques combined with mass spectrometry.

In another preferred embodiment, the expression levels of the *Sfrp1* gene or the SFRP1 mRNA are measured by a method selected from PCR, quantitative real time PCR (qPCR), digital PCR (dPCR), *Southern Blot,* RTLCR, RT-PCR, qRT-PCR, or any other method for the amplification of nucleic acids; DNA microarrays made with oligonucleotides deposited by any technique, DNA microarrays made with *in situ* synthesized oligonucleotides, *in situ* hybridization using specific labeled probes, electrophoresis gels, membrane transfer and hybridization with a specific probe, RMN or any other image diagnosis technique using paramagnetic nanoparticles or other type of functionalized nanoparticles, assays such as Western blot, immunoprecipitation assays, protein arrays preferably antibodies based microarrays, immunofluorescence, immunohistochemistry, ELISA or any other enzymatic method, by incubation with a specific ligand, or chromatographic techniques preferably combined with mass spectrometry. The presence and/or amount of products/substrates related to the activity of the SFRP1 protein may be measured in the screening method described above in order to detect the inhibition or blockage of the activity of said protein by the therapeutic agent being tested. Preferably, the products/substrates are an amyloid substrate/product or an analog thereof, more preferably the Aβ peptides. In a preferred embodiment, the products related to the activity of the SFRP1 protein that may be measured in step (b) of the screening method are Aβ1-42 peptides, sAPPα, ThioS APs, or accumulation of APs toxic forms. In another preferred embodiment, the activity of the ADAM10 secretase is measured in step (b) of the screening method disclosed above in order to detect the inhibition or blockage of the activity of the SFRP1 protein by the therapeutic agent being tested.

The "SFRP1 protein" mentioned in the present invention is, preferably, the human SFRP1 protein, more preferably wherein said human SFRP1 protein has the UniProtKB accession number Q8N474.

Another aspect of the invention refers to a pharmaceutical composition, hereinafter "the pharmaceutical composition of the invention", comprising an inhibitor of the SFRP1 protein or *Sfrp1* gene biological function, wherein the inhibitor is preferably the first monoclonal antibody (mAb) of the invention disclosed below. This pharmaceutical composition more preferably comprises the first and the second monoclonal antibodies of the invention disclosed below.

The "inhibitor of the SFRP1 protein or *Sfrp1* gene biological function" refers to any molecule that reduces the levels and/or the activity of the SFRP1 protein or the level to which the *Sfrp1* gene is transcribed in a cell. Examples of inhibitors are *Sfrp1* antisense sequences, preferably mRNA antisense sequences, siRNAs, anti-SFRP1 antibodies or antigen binding fragments thereof, SFRP1 agonists, soluble forms of the SFRP1 protein that act as competitors, or compounds discovered by the screening method disclosed herein, for instance, compounds or small molecules that bind the SFRP1 protein and neutralize its biological function. Examples of small molecules are peptides, peptidemimetics, amino acids, amino acid analogues, polynucleotides, polynucleotide analogues, organic or inorganic compounds, salts, and the like.

Preferably, the inhibitor of the SFRP1 protein or *Sfrp1* gene biological function referred to in the present invention is the small molecule WAY-316606 or at least one of the antibodies of the present invention disclosed herein. More preferably, the inhibitor of the SFRP1 protein or *Sfrp1* gene biological function referred to in the present invention and comprised in the pharmaceutical composition of the invention is the first mAb of the invention named "mAb 10.5.6" and disclosed below.

More preferably, this composition further comprises at least another active ingredient for the treatment of AD, even more preferably one or more anti-amyloid beta mAbs such as Bapineuzumab, Solanezumab, Gantenerumab, Crenezumab, BAN2401, Ponezumab and/or Aducanumab. In a more preferably embodiment, this anti-amyloid beta mAb is Aducanumab, which is commercially available.

In another preferred embodiment, the pharmaceutical composition of the invention further comprises a pharmaceutically acceptable vehicle.

The term "pharmaceutically acceptable vehicle" is a substance used in the composition to dilute any of the components comprised therein up to a certain volume or weight. The pharmaceutically acceptable vehicle is an inert substance or of identical action to any of the elements comprised in the composition of the present invention. The function of the vehicle is to facilitate the incorporation of other elements, to allow better dosing and administration or to give consistency and form to the composition. Additionally, the pharmaceutical composition of the invention may comprise one or more adjuvants and/or excipients. The term "excipient" makes reference to a substance that aids the absorption of the elements of the composition of the invention, stabilizes said elements, activates or aids the preparation of the composition in the sense of giving it consistency or providing flavours that make it more pleasant. Thus, the excipients could have the function of maintaining the ingredients bound together, such as for example in the case of starches, sugars or celluloses, the function of sweetening, the function of colouring, the function of protecting the composition, such as for example, to protect it from air and/or humidity, the function of filling a tablet, capsule or any other form of presentation, a disintegratory function for facilitating the dissolution of the components and its absorption in the intestine, without excluding other types of excipients not mentioned in this paragraph.

The term "adjuvant" refers to an agent that enhances the effect of the antibodies comprised in the composition when administered jointly with said antibodies. Adjuvants useful for the present invention are those commonly known in the art, such as Freund's complete or incomplete adjuvants, and the like.

Preferably, the composition of the invention comprises the inhibitors of the SFRP1 protein or *Sfrp1* gene biological function, preferably the mAbs of the invention, in a therapeutically effective amount, wherein a "therapeutically effective amount" is the level, amount or concentration of the inhibitors that produces the desired effect, improving cognitive performance, preferably, slowing down, treating and/or preventing AD, without causing adverse effects. The dose for obtaining a therapeutically effective amount depends on a variety of factors, such as for example, age, weight, sex or tolerance of the individual to whom the composition of the invention will be administered.

The composition may be formulated for its administration in a variety of forms known in the state of the art. Examples of preparations include any solid (tablets, pills, capsules, granules, etc.) or liquid (solutions, suspensions or emulsions) compositions, for oral, topical or parenteral administration. The composition of the present invention may also be in the form of sustained-release formulation of drugs or of any other conventional release system, such as nanoparticles, liposomes or nanospheres, a polymeric material, a biodegradable or non-biodegradable implant or biodegradable microparticles, such as for example, biodegradable microspheres. In another preferred embodiment, the composition of the invention is in the form of a solution for parenteral administration.

Such composition and/or its formulations may be administered to an animal and, therefore, to humans, in a variety of forms, including, but not limited to, intraperitoneal, intravenous, intradermal, intraspinal, instrastromal, intraarticular, intrasinovial, intrathecal, intralesional, intraarterial, intramuscular, intranasal, intracranial, subcutaneous, intraorbital, intracapsular, topical, by means of transdermal patches, percutaneous, nasal spray, surgical implant, internal surgical painting or infusion pump. The preferred administration form is intravenous.

Examples shown below evidence that the systemic injection of the first mAb of the invention through the retro-orbital sinus, or its intravenous administration, allows the entry of the mAb in the brain parenchyma. Thus, in a more preferred embodiment, the pharmaceutical composition of the invention is formulated for intravenous administration, as for example through retro-orbital sinus administration. Even more preferably, the composition is formulated for intravenous administration.

Another aspect of the invention refers to an inhibitor of the SFRP1 protein or *Sfrp1* gene biological function for use as a medicament. Another aspect of the invention, refers to an inhibitor of the SFRP1 protein or *Sfrp1* gene biological function for use in the treatment and/or prevention of AD. Preferably, the inhibitor referred to in these aspects of the invention is the small molecule WAY-316606 or at least one of the antibodies of the present invention disclosed herein, more preferably the first mAb of the invention.

The diagnostic and therapeutic applications referred to in the present invention may be extrapolated to any condition or pathology in which an accumulation of toxic Aβ peptides in the brain of a subject exists.

The medicament to which this invention refers may be administered to a subject in need thereof alone or in combination with one or more additional therapies and/or drugs for the treatment and/or prevention of AD, such as Aducanumab. This combined administration of the medicament of the invention may be simultaneous or sequential (at different time intervals).

The term "medicament" relates to any substance or combination of substances having properties for the treatment or prevention of diseases in organisms, preferably human beings, or that may be used or administered to the organisms, preferably human beings, with the aim of restoring, correcting or modifying damaged or impaired physiological conditions, by exercising a pharmacological, immunological or metabolic action. The "medicament" of the present invention may be for human or veterinary use, preferably for human use.

The term "treatment" relates to combating the effects caused as a consequence of the disease or pathological condition of interest, preferably AD, in a subject (preferably a mammal and, more preferably, a human) including:
(i) inhibit the disease or pathological condition, i.e. stop the development thereof;
(ii) alleviate the disease or pathological condition, i.e. cause the regression of the disease or pathological condition or its symptomatology;
(iii) stabilize the disease or pathological condition.

The term "prevention" consists of avoiding the onset of the disease, i.e. avoiding the disease or pathological condition in a subject (preferably a mammal and, more preferably, a human), in particular, when said subject has a predisposition for the pathological condition but has not been diagnosed yet.

### Monoclonal antibodies of the invention and their use for the treatment and/or prevention of AD

In the present invention, there have been developed some particular monoclonal antibodies that neutralize the SFRP1 protein activity. According to the examples shown below, the administration of an anti-SFRP1 treatment based on the specific anti-SFRP1 monoclonal antibodies disclosed herein prevented the formation and enlargement of toxic plaques in the brain of AD subjects.

Thus, another aspect of the invention refers to a monoclonal antibody, hereinafter "the first monoclonal antibody of the invention" or "the first mAb of the invention", that specifically binds the SFRP1 protein, wherein the light chain of the variable region of said monoclonal antibody comprises, preferably consists of, the amino acid sequence of SEQ ID NO: 1 and the heavy chain of the variable region of said monoclonal antibody comprises, preferably consists of, the amino acid sequence of SEQ ID NO: 2. This first mAb of the invention is also referred to in the present description as "mAb 10.5.6".

Preferably, the light chain of the variable region of the first mAb of the invention is encoded by a nucleotide sequence comprising, preferably consisting of, SEQ ID NO: 4 and the heavy chain of the variable region of the first mAb of the invention is encoded by a nucleotide sequence comprising, preferably consisting of, SEQ ID NO: 5.

Another aspect of the invention refers to a second monoclonal antibody, hereinafter "the second monoclonal antibody of the invention" or "the second mAb of the invention", that specifically binds the SFRP1 protein, wherein the heavy chain of the variable region of said second monoclonal antibody comprises, preferably consists of, the amino acid sequence of SEQ ID NO: 3. This second mAb of the invention is also referred to in the present description as "mAb 17.08.13".

Preferably, the heavy chain of the variable region of the second mAb of the invention is encoded by a nucleotide sequence comprising, preferably consisting of, SEQ ID NO: 6.

The term "antibody" relates to immunoglobulin molecules or to immunologically active portions of immunoglobulin molecules, i.e. molecules containing an antigen fixation site, which specifically bind (immune react) to the SFRP1 protein (the antigen). Examples of portions of immunologically active immunoglobulin molecules include fragments F(ab) and F(ab')2, which may be generated by treating the antibody with an enzyme such as pepsin or recombinantly. The antibodies mentioned in the present invention are, preferably, of IgG isotype, more preferably the first mAb of the invention is an IgG1 and the second mAb of the invention is an IgG2b.

The term "monoclonal antibody" refers to an antibody obtained from a population of substantially homogeneous antibodies, i.e., the individual antibodies comprising the population are identical except for possible naturally occurring mutations that are present in minor amounts. In some embodiments, monoclonal antibodies are made, for example, by the hybridoma method. In some embodiments, monoclonal antibodies are isolated from phage antibody libraries. In alternative embodiments, monoclonal antibodies are recombinantly generated. More preferably, the mAbs referred to in the present invention are generated by the hybridoma method.

The monoclonal antibodies described herein may be recombinant, chimeric, humanized, bi-specific, grafted, and/or fragments thereof, including antibodies altered by any means to be less immunogenic in humans. Thus, for example, the monoclonal antibodies and fragments thereof include "chimeric" antibodies and "humanized" antibodies.

In general, "chimeric antibodies" include a portion of the heavy and/or light chain that is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, so long as they exhibit the desired biological activity. For example, in some embodiments, a chimeric antibody contains variable regions derived from a mouse and constant regions derived from a human in which the constant region contains sequences homologous to human IgGs. Numerous methods for preparing "chimeric" antibodies are known in the art.

"Humanized" forms of non-human (e.g., murine) antibodies or fragments are chimeric immunoglobulins, immunoglobulin chains or fragments thereof (such as Fv, Fab, Fab', F(ab')2 or other antigen- binding subsequences of antibodies) which contain minimal sequences derived from a non-human immunoglobulin. Humanized antibodies include grafted antibodies or CDR grafted antibodies wherein part or all of the amino acid sequence of one or more complementarity determining regions (CDRs) derived from a non-human animal antibody is grafted to an appropriate position of a human antibody while maintaining the desired binding specificity and/or affinity of the original non-human antibody. In some embodiments, corresponding non-human residues replace Fv framework residues of the human immunoglobulin. In some embodiments, humanized antibodies comprise residues that are found neither in the recipient antibody nor in the imported CDR or framework sequences. These modifications are made to further refine and optimize antibody performance. In some embodiments, the humanized antibody comprises substantially all of at least one, and typically two, variable domains, in which all or substantially all of the CDR regions correspond to those of a non-human immunoglobulin and all or substantially all of the FR regions are those of a human immunoglobulin consensus sequence. Numerous methods for "humanizing" antibodies are known in the art.

In a preferred embodiment, the first mAb and/or the second mAb of the invention is humanized.

The expressions antibody "against the", "that reacts to", "specific for", "that specifically binds" or "that recognizes" the SFRP1 protein, can be used interchangeably. The specificity of the antibody for its antigen is the capacity of the same to bind the SFRP1 protein with a binding affinity that is preferably at least 2-fold, 50, 100 or 1,000-fold higher than its binding affinity for a nonspecific antigen different from the SFRP1 protein.

In another preferred embodiment, the first mAb and/or the second mAb of the invention is conjugated with a detection system and/or is immobilized in a support.

In another preferred embodiment, the first mAb of the invention and/or the second mAb of the invention is modified, by covalent or non-covalent reactions, with a substance that facilitates the penetration through the Blood Brain Barrier (BBB). The substance may be a BBB-penetrating peptide covalently bound to the C or N terminal of the mAb sequence and/or a release system, such as nanoparticles, liposomes or nanospheres.

In another preferred embodiment, the first mAb of the invention and/or the second mAb of the invention is coupled to an active principle, wherein more preferably the active principle is a drug.

As used herein, the term "active principle", "active substance", "pharmacologically active substance", "active ingredient" or "pharmacologically active ingredient" means any substance that provides a pharmacological activity in the mitigation, treatment and/or prevention of a pathological condition, preferably of AD. This term includes those components that lead to a chemical change during drug manufacturing. This term includes drugs and pro-drugs. In a more preferred embodiment, the active principle is for the treatment and/or prevention of AD.

In another aspect, the mAbs referred to in the present invention, preferably the first mAb of the invention coupled to an active principle, are used as a drug delivery system, preferably for the treatment and/or prevention of AD.

A "drug delivery system" refers to approaches, formulations, technologies, and systems for transporting an active principle in the body as needed to safely achieve its desired therapeutic effect in a site-specific manner. It involves site-targeting within the body, or it might involve facilitating systemic pharmacokinetics; in any case, it is concerned with both quantity and duration of drug presence.

Another aspect of the invention refers to the first mAb of the invention for use as a medicament. Alternatively, this aspect refers to the use of the first mAb of the invention for the manufacture of a medicament.

Another aspect refers to the first mAb of the invention for use in the treatment and/or prevention of AD. Alternatively, this aspect refers to the use of the first mAb of the invention for the manufacture of a medicament for the treatment and/or prevention of AD.

### Kit and in vitro method for the diagnosis of AD

Another aspect of the invention refers to the use of the expression levels of the *Sfrp1* gene as a quantitative biomarker for the *in vitro* diagnosis and/or prognosis of AD.

As shown in examples below, SFRP1 protein increase was paralleled by *Sfrp1* transcriptional up-regulation in AD patients. Thus, the term "expression levels of the *Sfrp1* gene" refers to expression levels of both a transcriptional or translational product of the gene, *i.e.* to mRNA or protein expression levels.

The term "quantitative biomarker" refers to the amount or concentration of mRNA or protein used as an indicator useful for the diagnosis and/or prognosis of AD.

In a preferred embodiment, the use of the expression levels of the *Sfrp1* gene as a quantitative biomarker for the *in vitro* diagnosis and/or prognosis of AD comprises the use of the first mAb of the invention. In a more preferred embodiment the first mAb of the invention is conjugated with a detection system and/or is immobilized in a support.

In a more preferred embodiment, the use of the expression levels of the *Sfrp1* gene as a quantitative biomarker for the *in vitro* diagnosis and/or prognosis of AD further comprises the use of the second mAb of the invention. In a more preferred embodiment the second mAb of the invention is conjugated with a detection system and/or is immobilized in a support.

The terms "labeled" or "conjugated with a detection system" are synonymous and mean that the antibody is conjugated with (linked to) a label. A "label" is any compound or molecule capable of giving rise to a detectable signal, preferably a chromogenic, fluorogenic, magnetic, electrodense, radioactive and/or chemiluminescent signal, allowing thus the detection of the labeled antibody. Labels that may be conjugated to an antibody are well known in the state of the art, for instance, a radioisotope [for example, 32P, 35S or 3H], a fluorescence or luminescent marker (fluorochrome) [for example, GFP or its derivatives, M-cherry, xanthenes such as fluorescein (FITC) or rhodamine, texas red, phycoerythrin (PE), bimanes, coumarins and their derivatives (e.g., umbelliferone and aminomethyl coumarins), alophycocyanin, 6-carboxyfluorescein (6-FAM), 2',7'-dimethyl-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), and the like], quantum dots, magnetic particles or electrodense particles (gold, ferritin, silica nanoparticles, etc.), a secondary antibody (labeled or not) or fragments thereof [for example, F(ab)2) fragments], an affinity label [for example, biotin, streptavidin, avidin, agarose, bone morphogenetic protein (BMP), haptens], an enzyme or a substrate of an enzyme [for example, alkaline phosphatase (AP), beta-galactosidase or horseradish peroxidase (HRP)], a dye [for example, Dylight 488, DyLight 405, DyLight 550, DyLight 650, DyLight 680, DyLight 755, DyLight 800, Alexa-Fluor dyes, APC, Cascade Blue, Cascade Yellow, Pacific Blue, PerCP, Cy5, Cy5.5, Cy7 or the like], or Amine-modified linker (S-HyNic, succinimidyl-6-hydrazino-nicotinamide linker; S-4FB, succinimidyl-4-formylbenzamide linker), aromatic amines (e.g., dansyl; squarate dyes), benzofurans, fluorescent cyanines, indocarbocyanines, carbazoles, or any combination thereof.

In a particular embodiment, the label or detection system to which the antibodies of the invention are conjugated is the enzyme HRP, more preferably the detection system of the invention is the system streptavidin-HRP.

In another particular embodiment, the monoclonal antibodies referred to in the present invention, preferably the second mAb of the invention, are biotinylated.

The label can be bound to the antibodies directly or it may be indirectly bound by means of another compound.

Labels are detected by any suitable method for detecting their signal. For example, a fluorescent label is detected by exciting the fluorochrome with the appropriate wavelength of light and detecting the resulting fluorescence, e.g., by microscopy, visual inspection, via photographic film, by the use of electronic detectors such as charge coupled devices (CCDs), or photomultipliers.

In some embodiments, all the antibodies referred to in the present invention are labeled with the same detection system. In some embodiments, the antibodies referred to in the present invention are labeled with different detection systems.

Furthermore, the antibodies may be linked to an affinity tag for their purification, preferably to a polyhistidine-tag, more preferably a 6xHis-Tag, in their N- or C-terminal ends.

The antibodies referred to in the present invention may also be immobilized on a support, for instance, a matrix surface (preferably a nylon matrix), a membrane; a glass, silicon, graphene or plastic support; a plate, preferably a microtiter plate; nanotechnology-based supports, particles, preferably nanoparticles; carbon derivatives and others, or on beads for example agarose spheres or superparamagnetic microspheres formed by biodegradable matrixes. The term "immobilized" means that the antibody is linked to any support or surface without losing its activity or antigen binding properties.

The attachment of the mAbs of the invention to a support facilities the implementation of the same in any standard equipment for the detection of antigen-antibody interactions.

Another aspect of the invention refers to a kit, hereinafter "the kit of the invention", for the diagnosis and/or prognosis of AD or cancer, preferably AD and more preferably in an isolated biological sample, that comprises the first mAb of the invention and/or the second mAb of the invention, preferably the first mAb of the invention. In a more preferred embodiment, it comprises the first and the second mAb of the invention.

Preferably, this kit is suitable for performing an ELISA assay, i. e. this kit preferably comprises all the elements needed for performing an ELISA assay for the detection and/or quantification of the amount of SFRP1 protein in a biological sample using the mAbs described herein.

In a preferred embodiment, the first mAb of the invention is conjugated with a detection system and/or is immobilized in a support in this kit of the invention. In another preferred embodiment, the second mAb of the invention is conjugated with a detection system and/or is immobilized in a support in this kit of the invention.

In some embodiments, the kit of the invention comprises a carrier, package, or container such as bottles, vials, syringes, and test tubes. In other embodiments, the containers are formed from a variety of materials such as glass or plastic.

In some embodiments, the kit of the invention comprises one or more additional containers, each with one or more of various materials (such as reagents, optionally in concentrated form, and/or devices) desirable from a commercial and user standpoint for use in the diagnosis and/or prognosis of AD or cancer, preferably AD, as described herein. Non-limiting examples of such materials include, but not limited to, buffers, solutions, primers, enzymes, diluents, filters, carrier, package, container, vial and/or tube, labels, listing contents and/or instructions for use and package. A set of instructions for performing an *in vitro* diagnosis and/or prognosis of AD or cancer, preferably AD, in a biological sample obtained from a subject is optionally included in the kit. In some embodiments, the containers comprised in the kit include an identification label. This identification label is preferably used to indicate that the contents of the kit are to be used for a specific therapeutic and/or diagnostic and/or prognostic application in AD. This identification label may also indicate guidelines for use of the contents of the kit, such as in the methods described herein.

The antibodies comprised in the kit may be free or immobilized, or both. More preferably, the antibodies are immobilized in a support comprised within the kit. For instance, the antibodies are immobilized in each of the wells of a microtiter plate comprised in the kit.

In another preferred embodiment, the kit of the invention further comprises a secondary antibody, which preferably binds to the first and/or second mAbs of the invention and more preferably is conjugated with a detection system. Detection systems to which this secondary antibody may be conjugated are the same as those previously indicated in this description. Preferably, this secondary antibody comprised in the kit of the invention is conjugated with the enzyme HRP.

In addition, the kit of the invention may optionally comprise a tertiary antibody that could be used in case the secondary antibody mentioned in the paragraph above is not conjugated. Preferably, this tertiary antibody binds to the secondary antibody comprised in the kit and more preferably is conjugated. Detection systems to which this tertiary antibody may be conjugated are the same as those previously indicated in this description. Preferably, this tertiary antibody comprised in the kit of the invention is conjugated with the enzyme HRP.

In a more preferred embodiment, the kit of the invention further comprises reactive sticks, fluorochrome/s, dye/s, substrate/s specific for the detection system selected to which the antibodies comprised in the kit are conjugated (these substrates are required for initiating the labeling reaction), blocking buffers, washing solutions and/or stop solutions.

"Substrate/s required for initiating the labeling reaction" are those compatible with the detection or conjugation system chosen. These substrates are those whose binding to their target (for instance, enzyme) triggers the reaction (for instance, enzymatic reaction) by which a detectable signal (for instance, chemiluminescence, fluorescence or colour) is produced. In another preferred embodiment, the substrate required for initiating the labeling reaction comprised in the kit of the invention is Tetramethylbenzidine (TMB) or O-phenylenediamine (OPD) or similar compounds. TMB is a substrate of the HRP enzyme. TMB is a chromogenic substrate used in staining procedures in immunohistochemistry as well as being a visualizing reagent used in enzyme-linked immunosorbent assays (ELISA). TMB is a white crystal powder that forms a pale blue-green liquid in solution with ethyl acetate. TMB acts as a hydrogen donor for the reduction of hydrogen peroxide to water by peroxidase enzymes such as HRP.

The "blocking buffer" or "blocking solution" optionally comprised in the kit of the invention is any buffer capable of blocking the protein sites not bound to an antibody with which it has been previously incubated. Blocking buffers that could be used in the present invention comprise non-fat dry milk, preferably together with PBS, StartingBlock, SuperBlock, BSA, Casein, BLOTTO (non-fat dry milk proteins in TBS), Pierce Clear Milk, Pierce Fast, Sea Block or Protein-Free.

The "washing solution" optionally comprised in the kit of the invention is any buffer capable of removing the unbound material to a support. The preferred washing solution used in the present invention is PBS containing Tween-20, although other washing solutions such as Tween-20 alone, PBS alone, TRIZMA BASE, Tris-HCI, TBS or Triton-X could also be used as alternatives or in combination with the preferred one.

The "stop solution" optionally comprised in the kit of the invention is any buffer capable of stopping the labeling reaction. The preferred stop solution used in the present invention is 3N H₂SO₄ or HCl 2N, more preferably 3N H₂SO₄.

The kit of the invention may also comprise all the elements needed for carrying out a diagnostic and/or prognostic method as described in the present invention. These additional elements may include, but without limitations, agents and/or proteins to prevent the contamination of the samples and/or elements comprised in the kit, or peptides and/or antibodies that act as positive or negative controls.

Another aspect of the invention refers to the use of the first mAb of the invention, the second mAb of the invention or the kit of the invention for the *in vitro* detection and/or quantification of the SFRP1 protein.

Another aspect of the invention refers to the use of a kit comprising antibodies, preferably monoclonal antibodies, specific for the SFRP1 protein in the *in vitro* diagnosis and/or prognosis of AD or cancer, preferably AD. In a preferred embodiment, this kit is the kit of the invention described above.

Another aspect of the invention refers to the use of the first mAb of the invention, the use of the second mAb of the invention or the use of the kit of the invention in the *in vitro* diagnosis and/or prognosis of AD or cancer, preferably AD, more preferably in the early diagnosis and/or prognosis of AD, even more preferably in an individual who is suspected of having or who is predisposed to suffering from AD.

The term "diagnosis" refers to the process performed in order to identify the presence or absence of a pathological condition, particularly AD or cancer, preferably AD, in a subject, preferably in a human subject. The term "prognosis" refers to the process performed in order to predict the events that will occur during the curse of a pathological condition, particularly AD or cancer, preferably AD, including the response to a treatment.

In a preferred embodiment, the diagnosis and/or prognosis of AD or cancer, preferably AD, referred to in the present invention are performed by immunoassay, more preferably by ELISA.

The ELISA assay referred to throughout the description and claims may be a direct ELISA, an indirect ELISA or a sandwich ELISA, or any combination thereof.

Another aspect of the invention refers to the use of the first mAb of the invention, and preferably also the second mAb of the invention, for the manufacture of a reactive for the diagnosis and/or prognosis of AD or cancer, preferably AD. Alternatively, this invention relates to the use of the first mAb of the invention, and preferably also the second mAb of the invention, as a reactive for the diagnosis and/or prognosis of AD or cancer, preferably AD.

Another aspect of the invention refers to an *in vitro* method for the diagnosis and/or prognosis of AD in a subject that comprises the following steps:
a) quantifying the level or amount of a product of the expression of the *Sfrp1* gene in a biological sample isolated from the subject,
b) comparing the value obtained in step (a) to a standard value obtained from the quantification of the expression level of the *Sfrp1* gene in a biological sample isolated from a healthy subject, and
c) assigning the subject of step (a) to the group of patients suffering from AD when the value obtained in step (a) is significantly higher than the standard value used for the comparison in step (b).

"A product of the expression the *Sfrp1* gene" may be mRNA or protein, preferably protein. Thus, preferably in step (a) the amount of SFRP1 protein is quantified. More preferably, the first mAb of the invention and/or the second mAb of the invention, or the kit of the invention is used for the quantification of step (a). Even more preferably, this method is performed by immunoassay, particularly by ELISA.

In a preferred embodiment of this method, the biological sample of step (a) is a brain sample or cerebrospinal fluid (CSF).

In another preferred embodiment, the subject is a mammal, more preferably a human.

In another aspect, the invention refers to a method for the *in vitro* diagnosis and/or prognosis of AD in a subject, preferably a human subject, that comprises the following steps:
a. Contacting an isolated biological sample obtained from the subject with the first mAb of the invention or with the kit of the invention (i. e. with the antibody/ies comprised in the kit of the invention),
b. incubating the mixture of step (a) under conditions that allow the binding of the antibody to its antigen (i. e. to the SFRP1 protein or a fragment thereof),
c. detecting and quantifying the amount of SFRP1 protein bound in the mixture of step (b),
d. comparing the amount obtained in step (c) to a standard value obtained from the quantification of the amount of SFRP1 protein in a biological sample isolated from a healthy subject, and
e. assigning the subject to the group of patients suffering from AD when the amount obtained in step (c) is significantly higher than the standard value.

This method, which will be also referred to as "the method of the invention", allows to diagnose AD in pre-symptomatic, mild and advance neuropathological stages of the disease. The diagnosis referred to in the present invention preferably refers to early diagnosis, i.e. prior to the onset of the AD symptoms.

The expression "quantifying" the amount of SFRP1 protein refers to the measurement of the amount or the concentration of protein. Techniques for measuring the amount of protein are, for example but without limitation, Western blot, MRI, flow cytometry, immunoprecipitation assays, protein arrays preferably antibodies based microarrays, immunofluorescence, immunohistochemistry, enzyme linked immunosorbent assays (ELISA) or any other enzymatic method, by incubation with a specific ligand, chromatographic techniques preferably combined with mass spectrometry, spectroscopy, mass spectrometry, colorimetry, electrophoresis or isoelectric focusing. For the immunoassay the antibodies used are preferably labeled and/or immobilized in a support as previously described in this description.

Preferably the detection and quantification of the amount of SFRP1 protein in step (c) is performed by *in situ* immunological hybridization. More preferably, steps (a) to (c) of this method of the invention are performed by immunoassay, preferably by ELISA.

An "immunoassay" or "immunochemical assay" is a biochemical assay that detects and/or quantifies the amount or quantity (preferably concentration) of one or more peptides (in the context of the present invention, the SFRP1 protein) of interest in a sample. For this purpose, the reaction uses one or more antibodies specific for the antigen/s to be detected. The quantification of the protein to be detected may be performed by any of the methods known in the art, such as the labeling of the antibody/ies or the antigen/s. The immunoassay may be competitive or non-competitive. In a competitive immunoassay the signal detected will be inversely proportional to the concentration of antigen in the sample. In a non-competitive assay (such as an "ELISA sandwich assay") the signal detected is directly proportional to the concentration of antigen in the sample. Examples of immunoassays are, but not limited to: immunoblotting (Western blot), enzyme-linked immunosorbent assay (ELISA), line immunoassay (LIA), radioimmunoassay (RIA), immunofluorescence, immunogold labeling (transmission electron microscopy), x-map or protein or lipopolysaccharide chips (LPS), real-time immunoquantitative PCR (iqPCR), electrochemiluminescent tags, label-free immunoassays (i.e. surface plasmon resonance, etc.), photoacoustic immunoassay, cloned enzyme donor immunoassay (CEDIA), lateral flow immunochromatographic assays, magnetic immunoassay (MIA), surround optical-fiber immunoassay (SOFIA), compact disk/digital versatile disk (CD/DVD) based immunoassay, or agglutination-PCR (ADAP).

ELISA is based on the assumption that an immunoreagent (antigen or antibody) can be immobilized onto a solid support, then this system is put in contact with a fluid phase containing the complementary reagent that can be bound to a marker compound (label). The signal produced as a consequence of the labeling reaction with the antibody conjugated with a detection system may be measured in the present invention, for instance but without limitation, by spectrophotometry, preferably at 450 nm, chemiluminiscence detection and quantification, spectrofluorometric detection and quantification, bioluminescence, differential calorimetry, analytical ultracentrifugation, interferometry, etc.

The term "amount" refers to the absolute or relative amount of protein.

The term "standard value" is considered to mean any value or range of values derived from the quantification of the SFRP1 protein in one or more biological samples isolated from healthy subjects.

By "healthy subject" is understood an individual not suffering from AD.

The group of subjects chosen for obtaining the standard value have the same or similar age as the subject under study and are representative of the population in which the method of the invention is to be applied. The quantification must be made in the same way and be obtained from the same type of isolated biological sample as that from the subject to be studied in step (a) of the method of the invention.

The term "comparing" as used in this invention refers, but is not limited to, the comparison of the amount of SFRP1 protein determined in the biological sample of step (a) with a standard value. The comparison described in step (d) of the method of the invention can be performed manually or computer-assisted.

An amount which is "significantly higher" than a standard value can be established by an expert in the field through the use of various statistical tools such as, for example but without limitation, by determination of confidence intervals, determination of the p value, two-tailed unpaired Student's t test, Fisher's discriminant functions, using Kruskal-Wallis one way analysis with Dunn's multiple comparisons test, one-way ANOVA with Bonferroni's post hoc multiple comparisons test, two tailed Mann-Whitney U test or Kruskal-Wallis U tests.

Preferably, the biological samples referred to in this invention are isolated from human beings.

The term "isolated biological sample" refers to any sample obtained from any tissue or fluid of a subject, which may be obtained by any method known in the art. The biological sample may be a tissue or fluid sample obtained preferably from the brain. Alternatively, it may be a blood, plasma, serum, lymph, urine, cerebrospinal fluid, mucus, sputum, saliva, sweat, isolated cells, biopsy sample, tissue homogenates, etc., sample.

In another preferred embodiment, the isolated biological sample is a brain sample, preferably a brain cortex or cortical sample, or cerebrospinal fluid (CSF), more preferably the biological sample is CSF. In another preferred embodiment, the isolated biological sample is an entorhinal cortex sample, but other brain region samples are also useful.

The term "cerebrospinal fluid" refers to a liquid of transparent colour that bathes the brain and spinal cord. Many diseases change its composition and its study is important and frequently determining for the diseases of the central and peripheral nervous system. Included in these diseases are the neurodegenerative diseases such as Alzheimer's or Parkinson's disease.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by the practice of the invention. The following examples, drawings and sequence listing are provided by way of illustration and are not intended to be limiting of the present invention.

### DESCRIPTION OF THE DRAWINGS

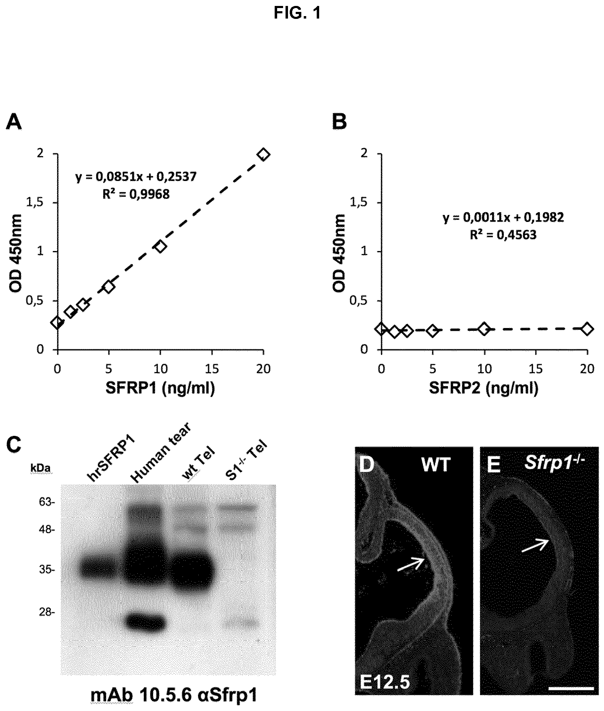

**Fig. 1****. Characterization of anti-SFRP1 monoclonal antibodies.** (**A, B**) Representative ELISA standard curve showing the specificity of the assay that recognizes recombinant hSFRP1 **(A)** but not the highly related hSFRP2 **(B). (C)** Western blot analysis showing the specificity of mAb 10.5.6. Lane 1: human recombinant SFRP1, lane 2: human tear (a fluid enriched in SFRP1); lane 3: extract from E12.5 wild type embryonic telencephalon; lane 4 extract from the telencephalon of Sfrp1^{-/-} E12.5 embryos. The mAb recognizes both human and mouse tissue. **(D, E)** Frontal cryostat sections of the telencephalon from wt (D) and *Sfrp1*^{*-*/*-*}(E) E12.5 embryos immunostained with the 10.5.6 mAb. Sections were counterstained with Hoechst. There is absence of staining in the *Sfrp1*^{*-*/*-*} neuroepithelium (residual signal in the ectoderm is unspecific, E). Scale bar: 100 µm.

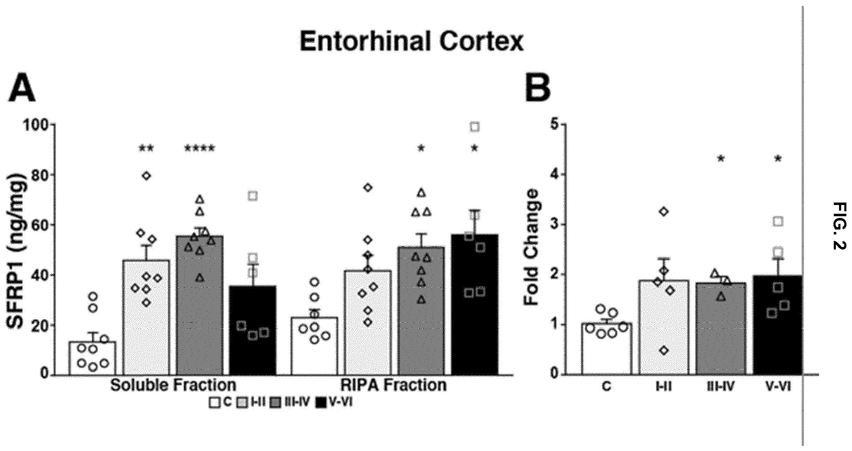

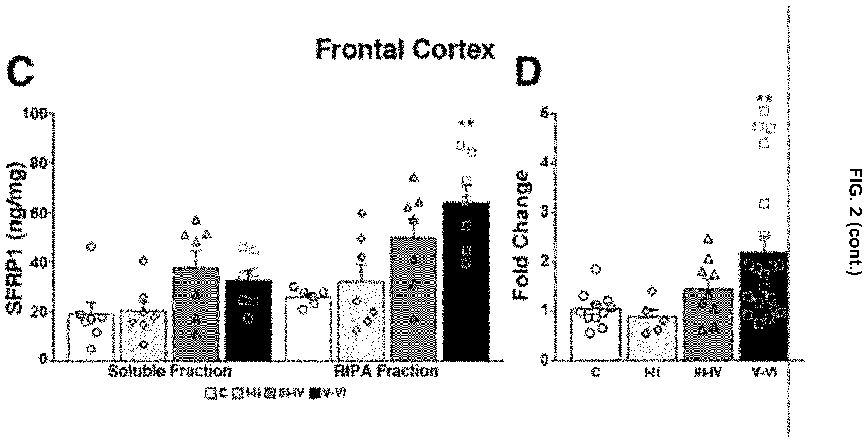

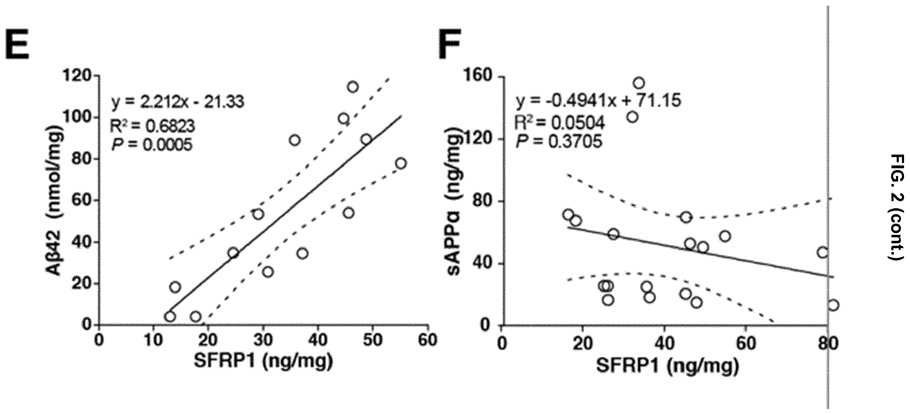

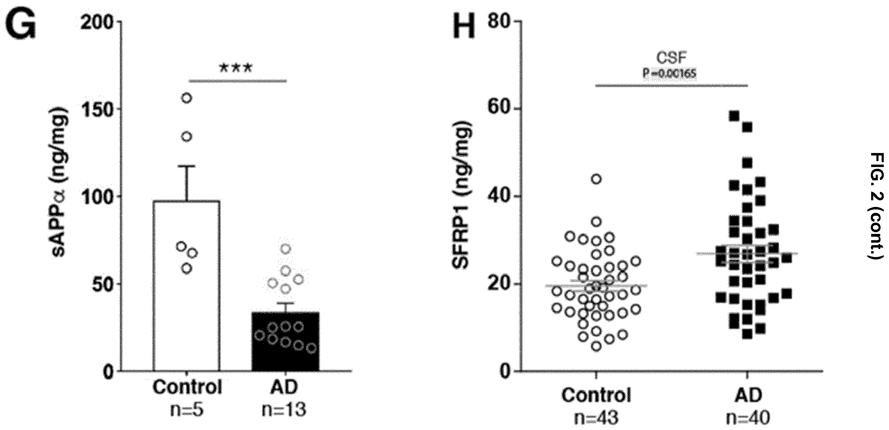

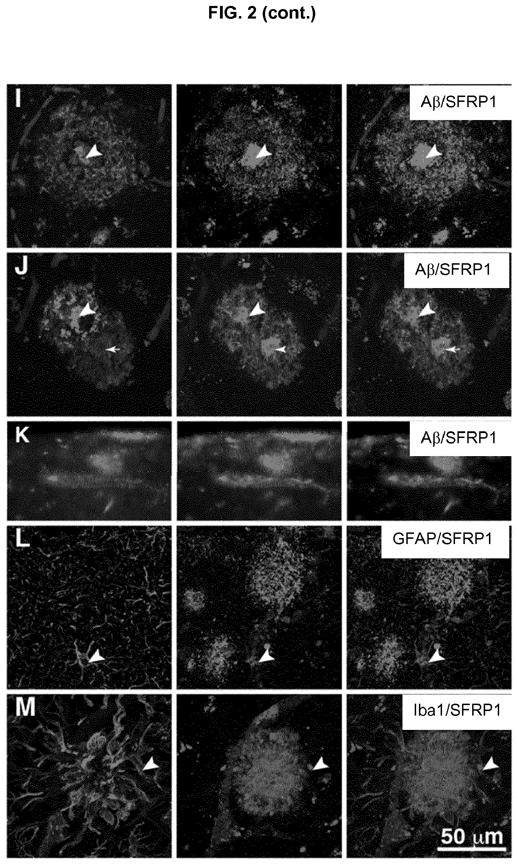

**Fig. 2****. SFRP1 is upregulated in human samples from AD patients correlating with pro-amyloidogenic APP processing.** (**A, C**) The graphs show ELISA determination of SFRP1 protein levels in TBS and RIPA-soluble fractions of entorhinal and frontal cortex from controls (C) and AD patients at different BB stages as indicated in the graphs. Data were analysed with two-ways ANOVA followed by Bonferroni test. *p< 0.05; ***p*≤0.01; ****p*≤0.001; *****p*<0.0001. **(B, D)** The graphs show fold enrichment of *SFRP1* mRNA levels in the entorhinal and frontal cortex of AD at different BB stages when compared with controls. Values were determined by Taqman PCR assays, normalized against the levels of HPRT and AARS housekeeping genes. Results were analysed with Kruskal-Wallis followed by post hoc Mann-Whitney test. Differences between groups were considered statistically significant at p-value: * *p*≤0.05; ***p*≤0.01. **(E, F)** Scatter plot of Aβ42 **(E)** and sAPPα **(F)** versus SFRP1 levels in samples of human entorhinal cortex. SFRP1, Aβ42 and sAPPα levels were determined by specific ELISA in the same samples. Note the significant positive correlation between SFRP1 and Aβ42 levels (**E**) and the tendency towards a negative correlation between SFRP1 and sAPPα (**F**). **G**) The graph shows ELISA based determination of sAPPα levels in human control and AD entorhinal cortex extracts. **H**) The graph shows SFRP1 levels in CSF samples from cohorts of AD patients and age-matched controls (Table S2). Data were analysed with T-test, significance is indicated in the graph. **(I-M)** Confocal images of frozen sections from the frontal cortex of AD patients (BBV-VI) co-immunostained with antibodies against SFRP1 (red) and Aβ (clone 6E10) **(I-K)**, GFAP (**L**) or Iba1 (**M**) (all in green). In each row, single and merged channels are shown. Note that SFRP1 accumulates in the core of the Aβ+ plaque (arrowheads in **I**, **J**), forms aggregates in the AP halo (arrow in **J**), is associated to blood vessels (**K**) and localizes to GFAP+ (arrowhead in **L**) and Iba1+ cells (arrowhead in **M**). Scale bar: 50µm.

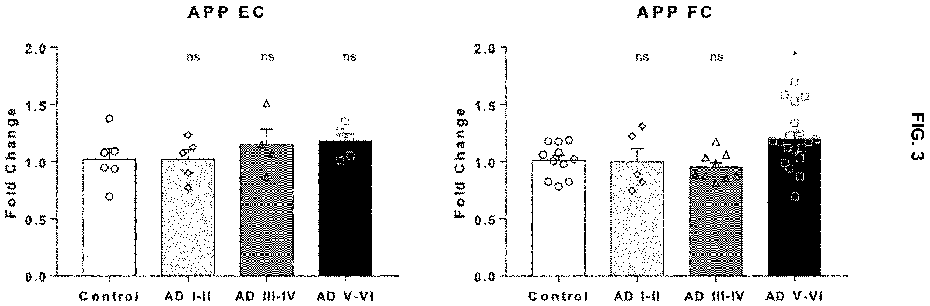

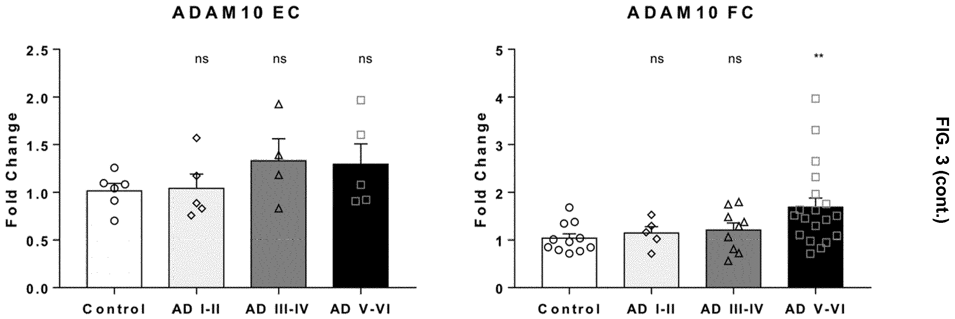

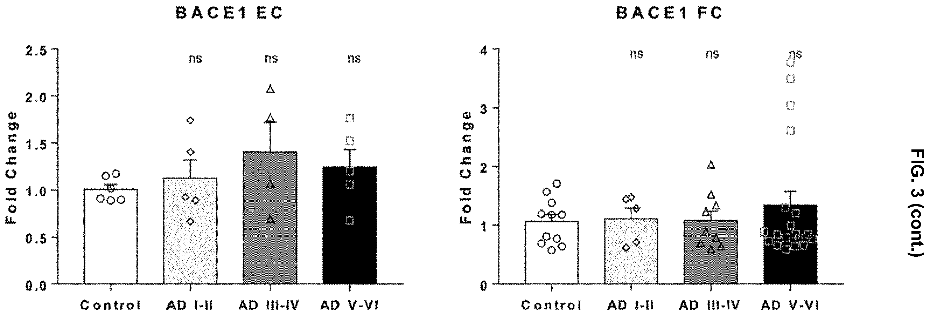

**Fig. 3****. Expression of *APP, ADAM10,* and *BACE1* in AD human cortical samples.** The graphs show fold enrichment of *APP, ADAM10* and *BACE1* mRNA levels in the entorhinal (EC) and frontal (FC) cortex from control and AD III-IV/0-C (III-IV) and AD V-VI/B-C (V-VI) samples. Values were determined by Taqman PCR assays, normalized against the levels of HPRT and AARS housekeeping genes. There is only a significant increase of APP and ADAM10 levels at late stages of the disease when compared to control samples. Results were analyzed with Kruskal-Wallis followed by post hoc Mann-Whitney test. Differences between groups were considered statistically significant at p-value: * p≤0.05; n.s., non-significant.

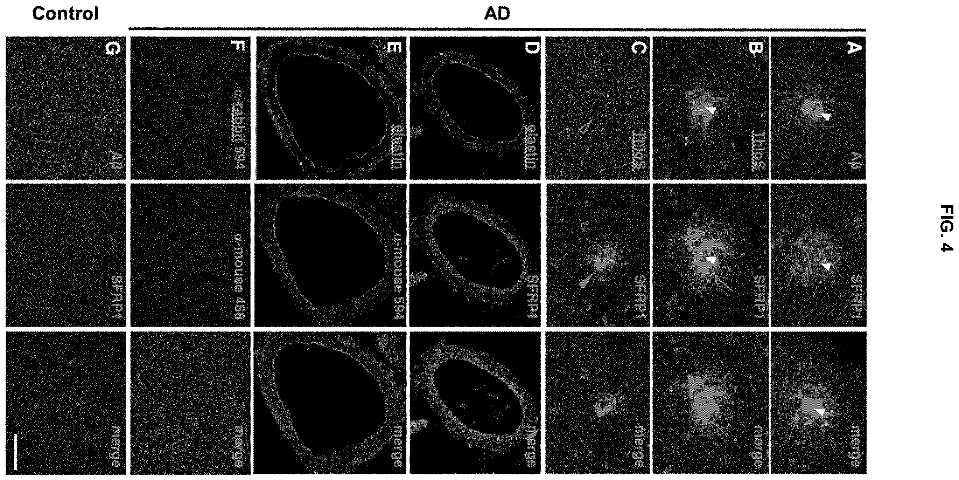

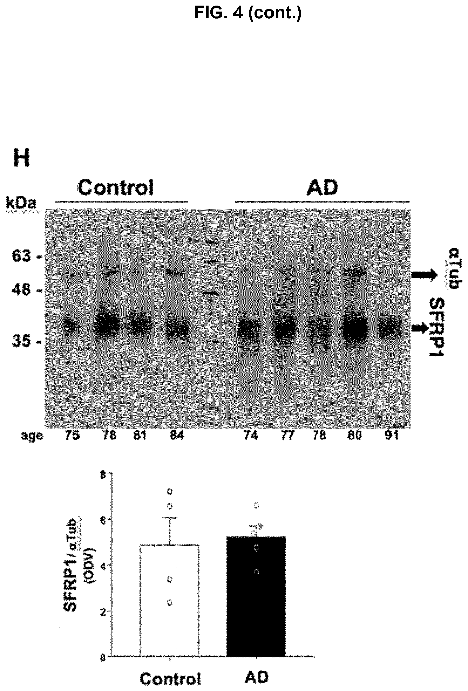

**Fig. 4****. SFRP1 specifically localizes to APs, blood vessels and choroid plexus.** (**A-G**) Paraffin sections of the frontal cortex from BBV-VI AD patients (**A-F**) and age matched controls (**G**) coimmunostained for SFRP1 and Aβ, or ThioS or probed with secondary antibodies only, as indicated in the panels. Note that SFRP1 specifically accumulates in the core of Aβ+ and ThioS+ APs (white arrowheads) and their surroundings (yellow arrows in **A,B**) and forms aggregates that are poorly ThioS+ (arrowhead in **C**). SFRP1 also specifically localizes to blood vessels identified by the auto-fluorescence of elastin **(D,E)**. **(F)** No signal was detected with secondary antibodies, supporting the specificity of SFRP1 localization. **G**) No detectable signal was found in control sections. **H)** Western blot analysis of SFRP1 presence in choroid plexus extracts from AD patients and cognitive normal controls, normalized to a-Tubulin. Data were analyzed with Student-t test. Scale bar: 50 µm A-C; 100 µm D-G.

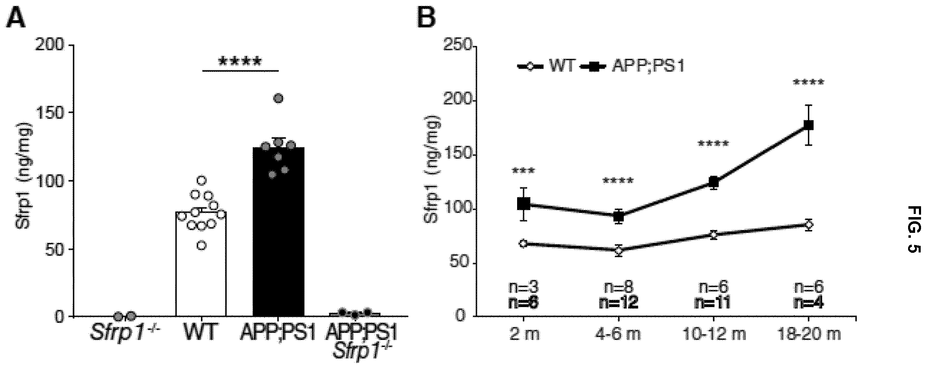

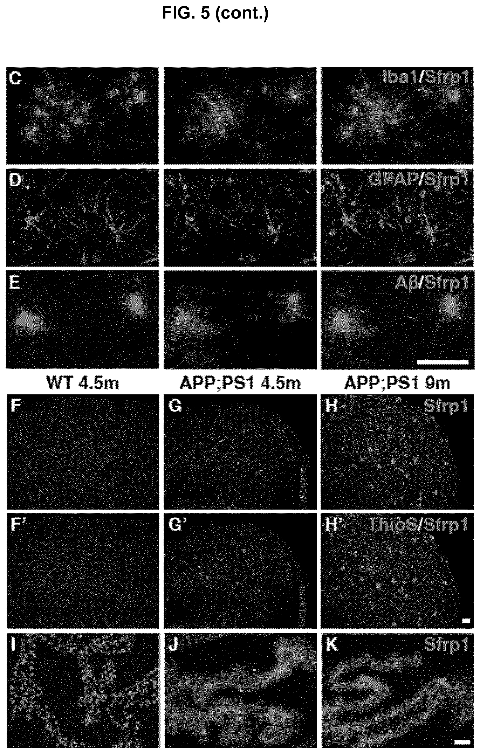

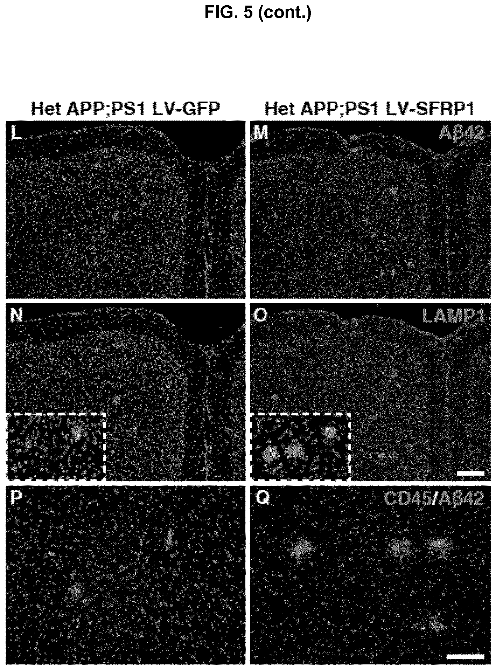

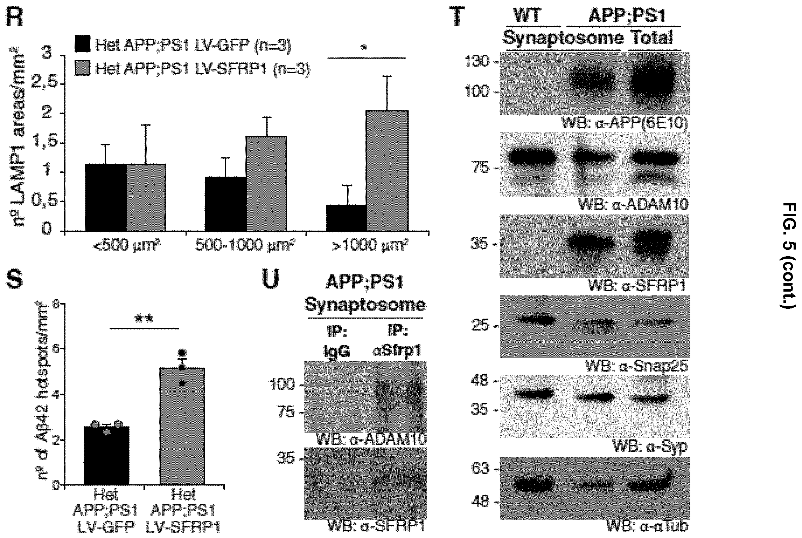

**Fig. 5****. Progressive upregulation of brain Sfrp1 correlates with the age of APP;PS1 mice brains and its forced expression accelerates the appearance of APs.** (**A**) ELISA determination of Sfrp1 levels in brain extracts from 10-12 months old mice of the indicated genotypes. (**B**) ELISA determination of SFRP1 protein levels in brain extracts of wt APP;PS1 and *Sfrp1*^{-/-} and APP;PS1;*Sfrp1*^{-/-} (used as negative controls) mice at different ages. (**C-K**) Frontal cortical sections from 4.5 months-old APP;PS1 brains co-immunostained as indicated in each panel. Sfrp1 co-localizes with some Iba1+ microglial cells (**C**), GFAP+ astrocytes (**D**) and Aβ+ (**E**) and ThioS+ AP (**F**). (**G-L)** Frontal cryostat sections from the mouse cortex (age and genotype indicated in panel) immunostained for Sfrp1. Positive signal localizes to the APs and choroid plexus of APP;PS1 mice but is poorly detected in wt. (**M-Q**) Frontal cryostat sections from the cortex of 5.5 months-old heterozygous APP;PS1 transduced at 2.5 months of age with lentiviruses (LV) carrying *GFP* (**L,N,P**) or *Sfrp1*-IRES-GFP (**M,O,Q**). Sections were co-immunostained for Aβ42, LAMP1 (**N-O**) or the activated microglial marker CD45 (**P,Q**). (**R,S**) Quantification of LAMP1+ area (R) and Aβ42+ hotspots (S) in APP;PS1 transduced animals. Data are means ±s.e.m. The number (n) of quantified animals is indicated in the bars. Data were analyzed with Student-t test**p*≤0.05; ***p*≤0.01. **U**) Western blot analysis of Co-IP studies showing that Sfrp1 localizes at the synapses of APP;PS1 animals and interact with APP. (**T**) Western blot analysis showing the presence of APP, Sfrp1, ADAM10 in the Snap25+ synaptic fractions. Scale bars: 100 µm.

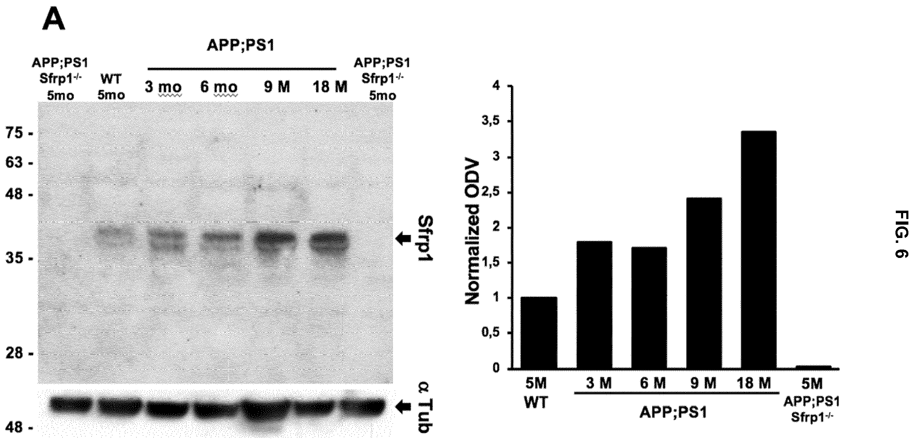

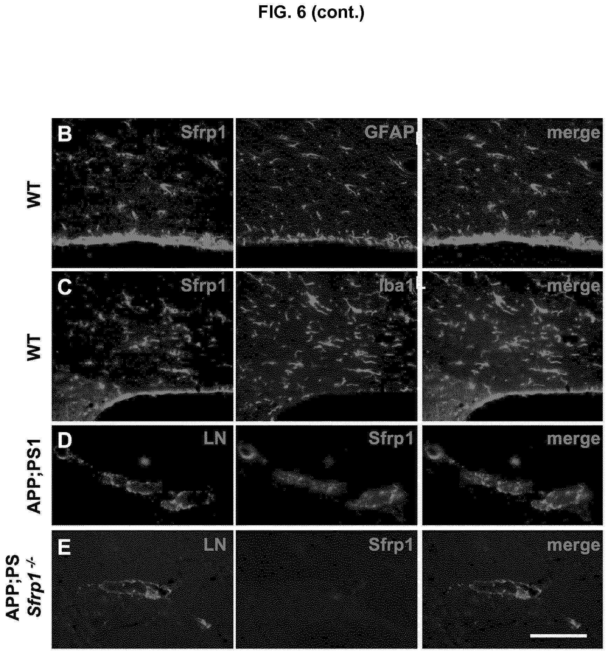

**Fig. 6****. Distribution of Sfrp1 in wt and APP;PS1 mouse cortex. A**) Western blot analysis of SFRP1 expression in cortical extracts from wt and APP;PS1 animals at the ages indicated in the panels. SFRP1 expression increases with the progression of the disease in the AD mice, as shown by densitometry of the bands (graph on the right). (**B-E**) Frontal cryostat sections of the cortex from 4.5-months-old wt. APP;PS1 and APP;PS1;*Sfrp1*^{-/-} mice co-immunostained for SFRP1 and GFAP or Iba1 or laminin (LN), as indicated in the panels. In mice, Sfrp1 localizes to the walls of the lateral ventricle overlapping with some GFAP+ and Iba1+ cells. SFRP1 is also found in LN+ blood vessels. Staining in the vessels is not found in mice lacking *Sfrp1* (F). Scale bars: 100 µm.

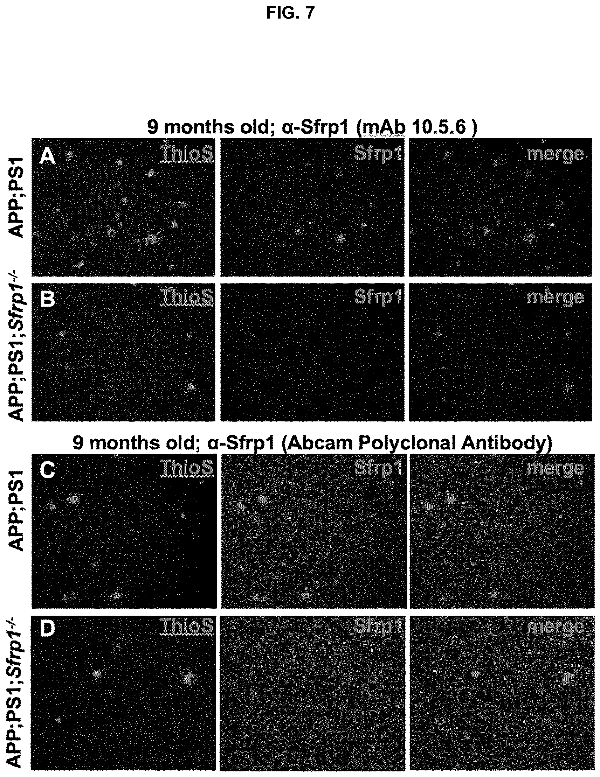

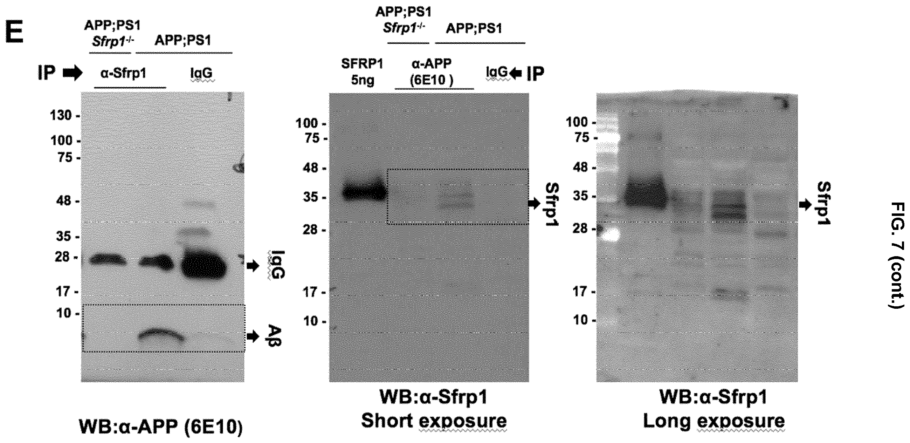

**Fig. 7****. Sfrp1 specifically localizes to APs and interacts with Aβ in APP;PS1 mice. A-D**) Cryostat sections from the cortex of 9-months-old APP;PS1 and APP;PS1;*Sfrp1*^{-/-} mice immunostained with 10.5.6 mAb or with a rabbit polyclonal (Abcam ab4193) against Sfrp1 and counterstained with ThioS, as indicated in the panels. Sfrp1 localizes to ThioS+ plaques in APP;PS1 mice with both antibodies. No Sfrp1 + signal was observed in the few ThioS+ APs present in APP;PS1;*Sfrp1*^{-/-} mice strongly supporting the specificity of the signal in the AP. **E)** Western blot analysis of Aβ and Sfrp1 co-immunoprecipitations from cortical extracts of APP;PS1 and APP;PS1;*Sfrp1*^{-/-} mice as indicated in the panels. Scale bar: 200 µm

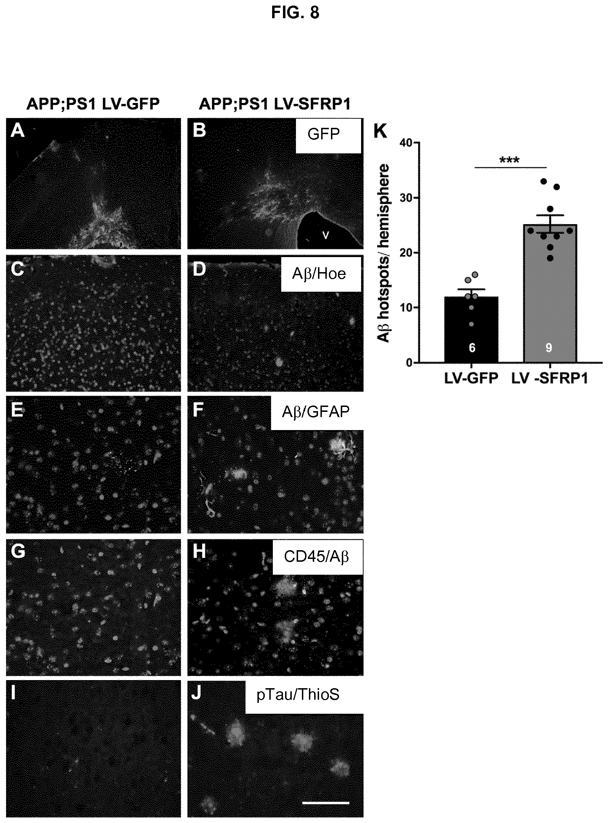

**Fig. 8****. Forced expression of *Sfrp1* accelerates the appearance of APs. A-J)** Frontal sections of the cortex from 3 months-old APP;PS1 mice transduced one month earlier with lentiviral particles (LV) expressing GFP or Sfrp1-IRES-GFP as indicated in the panel. Sections in A, B shows anti-GFP immunostaining reflecting the LV infection close to the lateral ventricle (v) and extending dorsally to the cortex. Sections in C-J were co-immunostained for Aβ and GFAP or CD45, or stained with ThioS and immunostained for p-Tau, as indicated in the panels. Transduction with *Sfrp1*-LV promotes the formation of AP and of the associated reactive gliosis. (**K**) The graph shows quantification of the number of APs found in the cortex of infected mice. Scale bar: 100 µm.

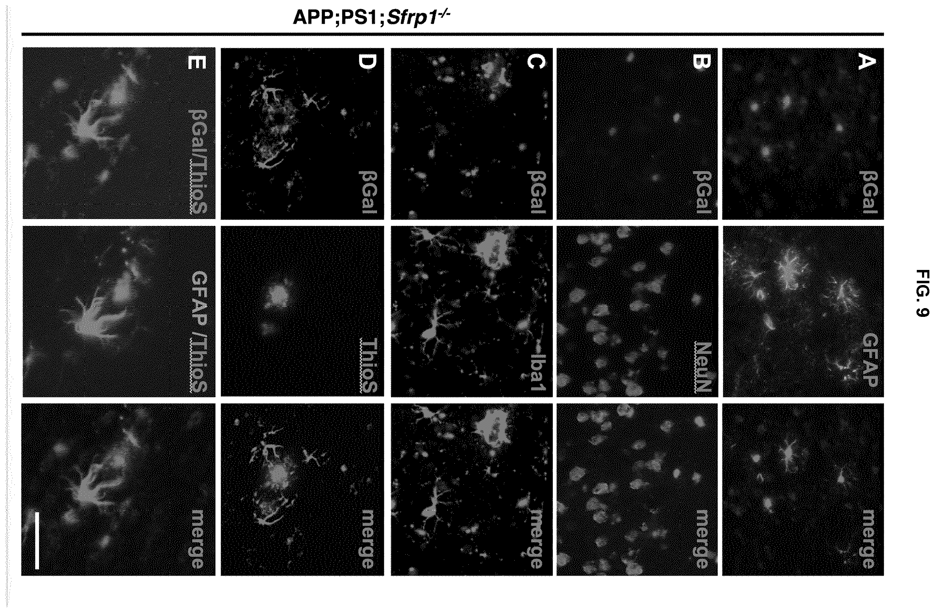

**Fig. 9****. Astrocytes and microglial cells are the source of brain Sfrp1. (A-E)** Cryostat sections of the cortex from 4.5-months-old APP;PS1; *Sfrp1*^{-/-} mice co-immunostained with antibodies against nuclear β-Gal and GFAP, NeuN or Iba1, or stained with ThioS, as indicated in the panels. The nuclear β-Gal, reflecting *Sfrp1* expression, localizes to GFAP and Iba1 positive cells, but no to NeuN positive cells. There is a strong β-Gal signal in cells close to ThioS+ plaques. Scale bar: 100 µm.

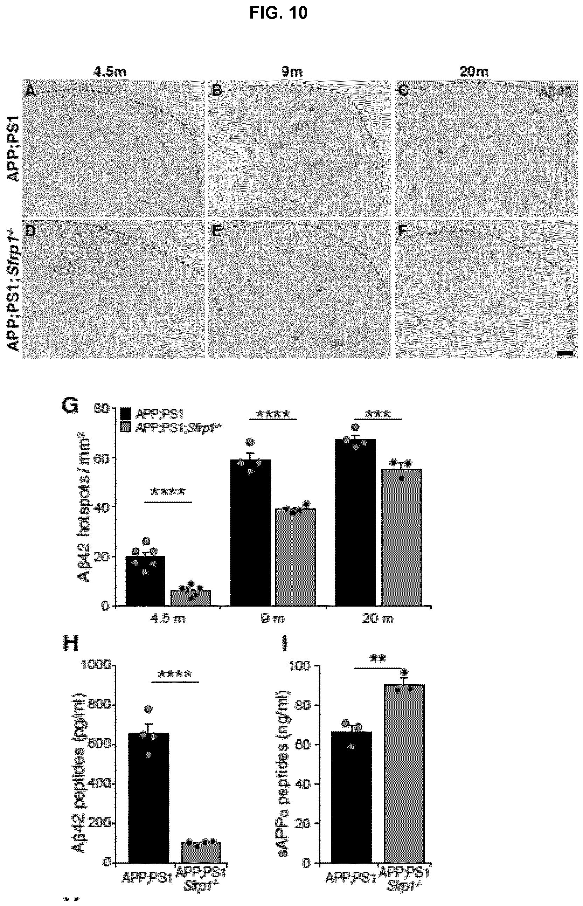

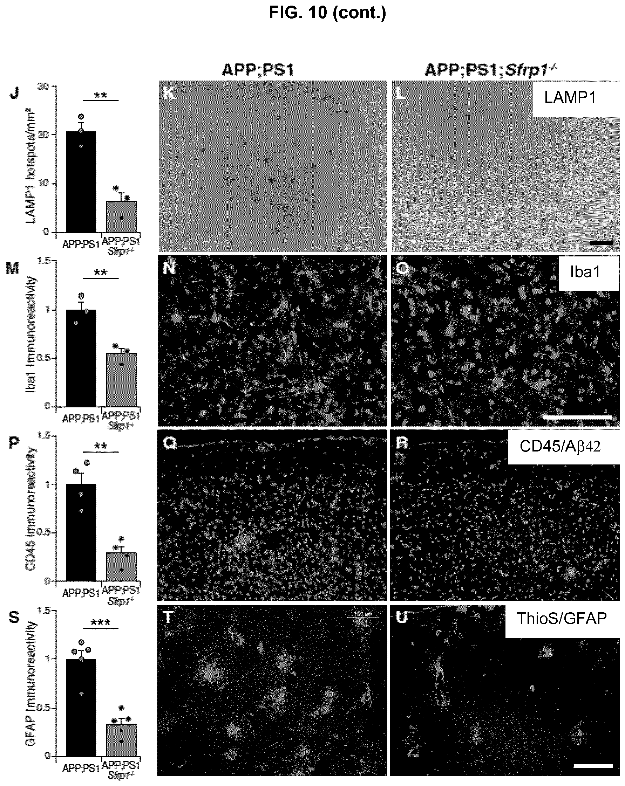

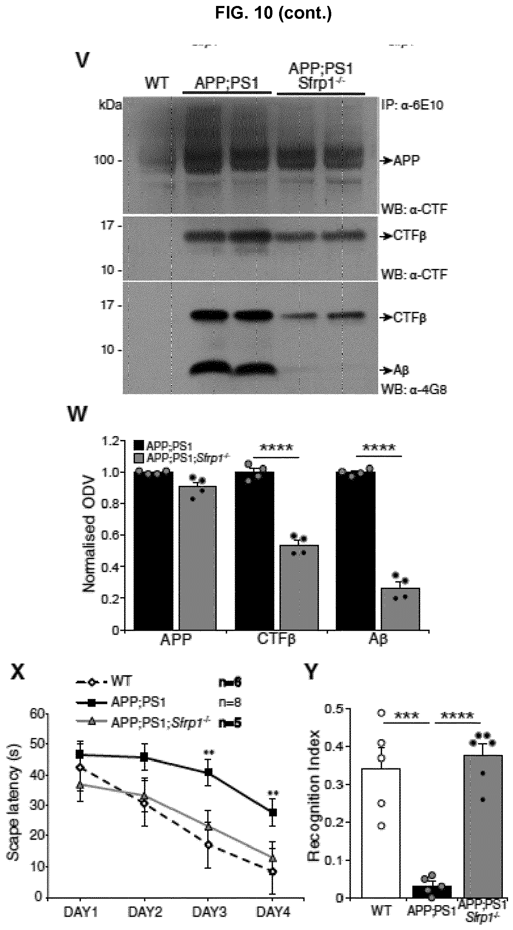

**Fig. 10****. *Sfrp1* inactivation prevents the appearance of pathological traits in APP;PS1 mice. (A-F)** Frontal cryostat sections from the cortex of 4.5, 9 and 20 months-old APP;PS1 and APP;PS1;*Sfrp1*^{-/-} mice immunostained for Aβ42, as indicated in the panels. **G**) Quantification of Aβ42 hotspots illustrated in A-F. Data were analyzed with two-way ANOVA followed by Bonferroni test. **H, I**) The graphs show ELISA based determination of Aβ42 and sAPPα levels present in cortical extracts from 6 months-old APP;PS1 and APP;PS1;*Sfrp1*^{-/-} mice. Data were analyzed with Student-t test. **J-U**) Frontal cryostat sections of the cortex from 4.5 months-old APP;PS1 and APP;PS1;*Sfrp1*^{-/-} mice immunostained as indicated in the panels and relative quantification of immune-positive signals. Data were analyzed with Student-t test. **V)** Western blot analysis of TBS-TX100 extracts from 6 months-old APP;PS1 and APP;PS1;*Sfrp1*^{-/-} brains immunoprecipitated (IP) with anti-Aβ (6E10) antibodies and visualized with anti-APP C-terminal (anti-CTF) and anti-Aβ (4G8) antibodies (extended information, Fig S13). (**W**) Quantification of immunoreactive bands as those shown in W normalized to FL APP levels. Data were analyzed with two-way ANOVA followed by Bonferroni test. (**X, Y**) The graphs depict temporal learning skills (**X**), and recognition index (**Y**) of wt, APP;PS1 and APP;PS1;*Sfrp1*^{-/-} 8 months-old mice subjected to Morris water maze **(X)** and ORT **(Y)**. Data were analyzed with one- or two-way ANOVA followed by Tukey method (**X,Y**). **p*≤0.05; ***p*≤0.01; ****p*≤0.001; *****p*≤0.0001. Scale bars: 100 µm

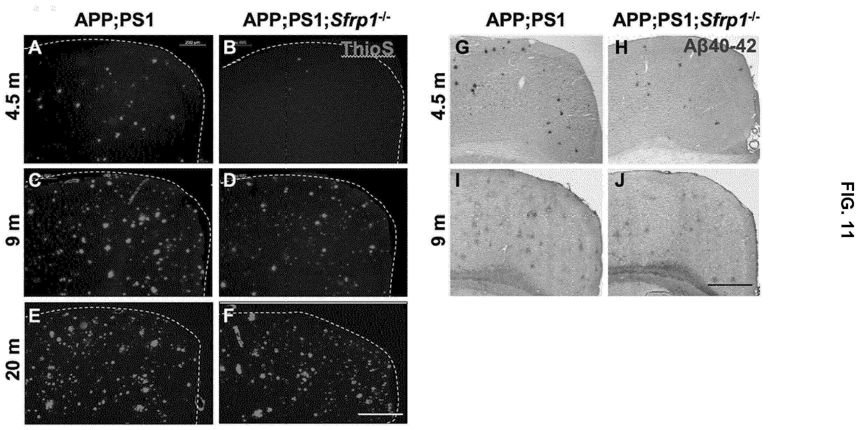

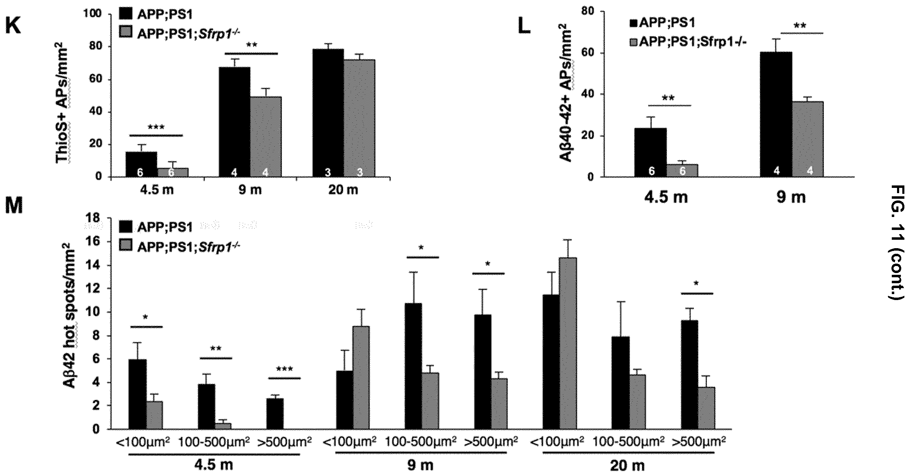

**Fig. 11****. Sfrp1 inactivation critically delays the appearance of amyloid plaques in APP;PS1 mice. A-F)** Frontal cryostat sections of the cortex from 4.5, 9 and 20 months-old APP;PS1 and APP;PS1;*Sfrp1*^{-/-} mice stained with ThioS as indicated in the panels. (**G-J**) Frontal sections of the cortex from a different set of 4.5 and 9 months-old APP;PS1 and APP;PS1;*Sfrp1*^{-/-} mice stained with antibodies against Aβ40-42. **K**) Quantification of the number of ThioS+ APs in 4.5, 9 and 20 months-old APP;PS1 and APP;PS1;*Sfrp1*^{-/-} mice. **L**) Quantification of the number of Aβ40-42+ APs in APP;PS1 and APP;PS1;*Sfrp1*^{-/-} mice. **M**) Quantification of the size distribution of Aβ+ APs illustrated **in** **Fig 3A-F**. Note the significant decrease in the size of Aβ+ hotspots in the absence of *Sfrp1.* Data were analyzed with Student-t test. Number of analyzed animals is indicated in the bars. Scale bar: 100 µm

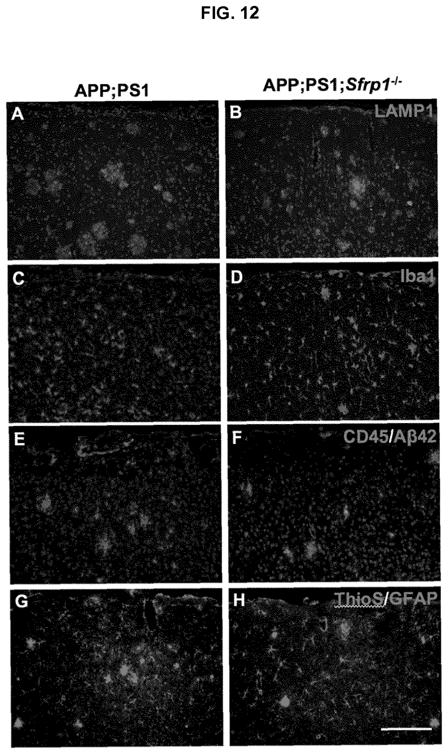

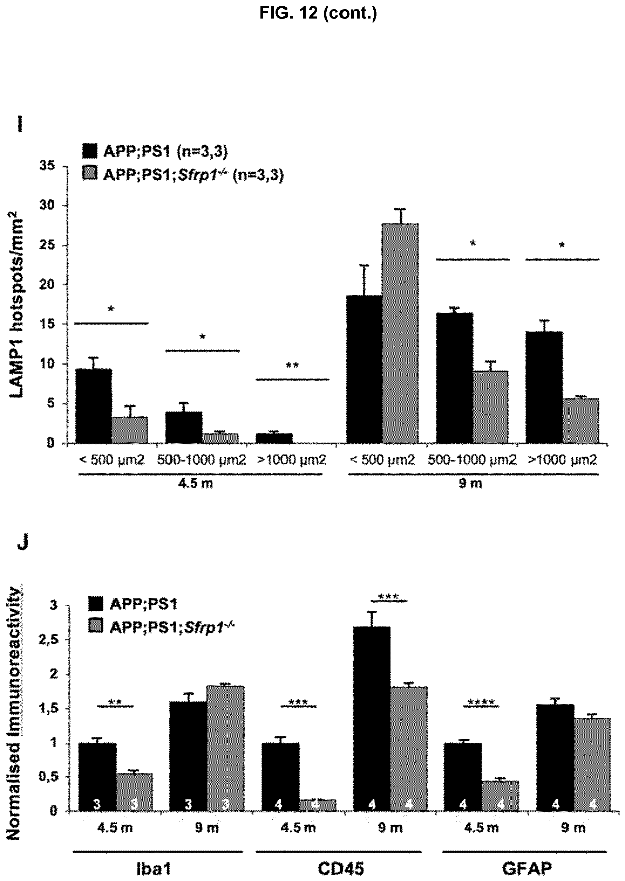

**Fig. 12****. Activation of glial cells is reduced in APP;PS1;*Sfrp1*^{-/-} mice. A-H)** Frontal sections of the cortex from 9 months-old APP;PS1 and APP;PS1;*Sfrp1*^{-/-} mice immunostained with antibodies against LAMP1, Iba1 CD45 and Aβ42 or GFAP and stained with ThioS, as indicated in the panels. (**I**, **J**) Quantification of LAMP1 hotspots and Iba1, CD45 and GFAP immunoreactivity in 4.5 and 9 months old cortex of APP;PS1 and APP;PS1;*Sfrp1*^{-/-} mice. Scale bars: 100 µm.

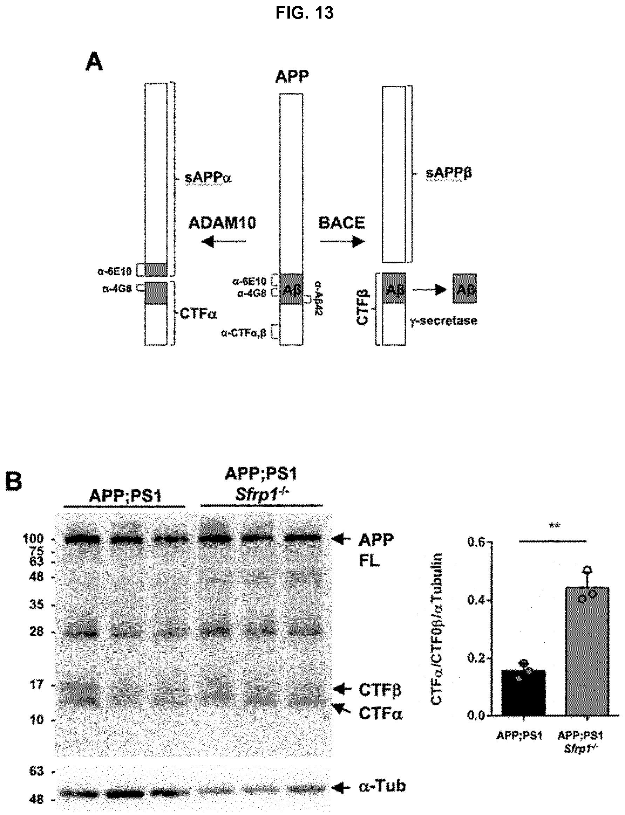

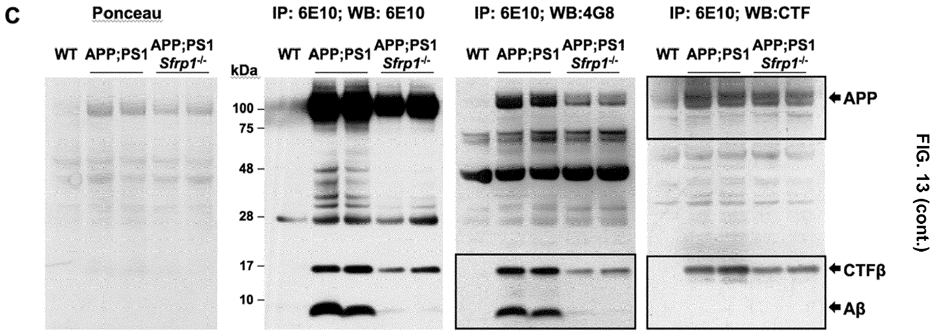

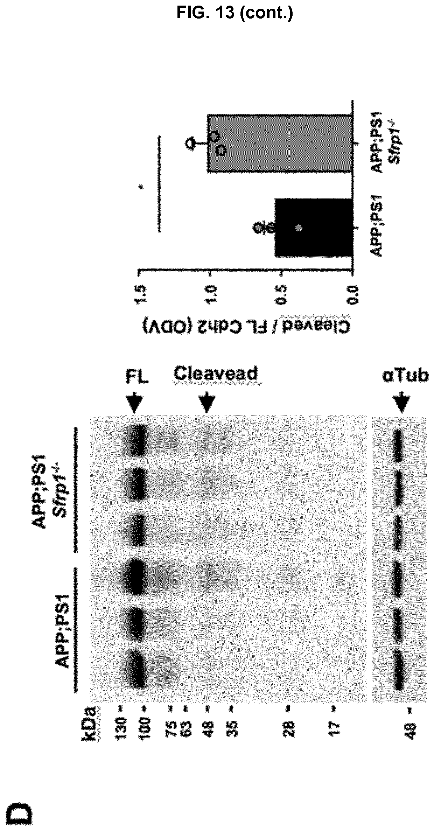

**Fig. 13****. ADAM10 activity is increased in the absence of Sfrp1. (A)** Schematic representation of the APP molecule to indicate which are the fragments recognized by each one of the antibodies against APP used in this study. The Aβ peptide is depicted in red. **B**) Western blot analysis of CTFα, CTFβ and APP content in cortical extracts from APP;PS1 and APP;PS1;Sfrp1-/- mice and their quantification. The membrane was probed with the anti-APP C-ter antibody. **C**) Uncropped Ponceau nitrocellulose membrane staining and WB images displayed in Fig. 3V. **D**) Western blot analysis of N-cadherin (Cdh2) processing in APP;PS1 and APP;PS1;*Sfrp1*^{-/-} brains. Values were normalized to α-Tubulin levels used as a loading control. Processing was determined by the levels of cleaved vs full length levels of the protein. Note the significant increase in Cdh2 processing in the absence of Sfrp1, indicating a higher ADAM10 activity. Data were analyzed with Student-t test*p≤0.05.

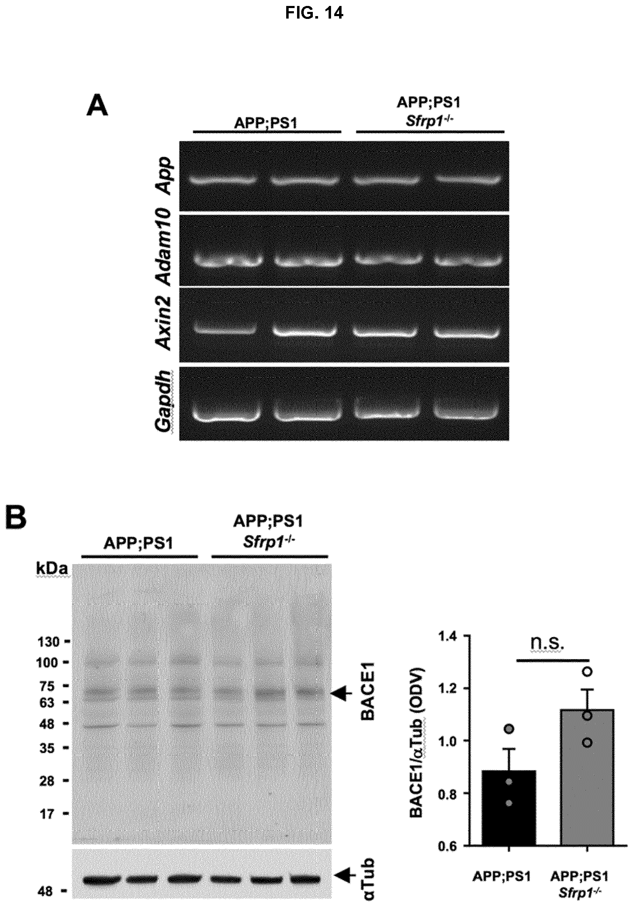

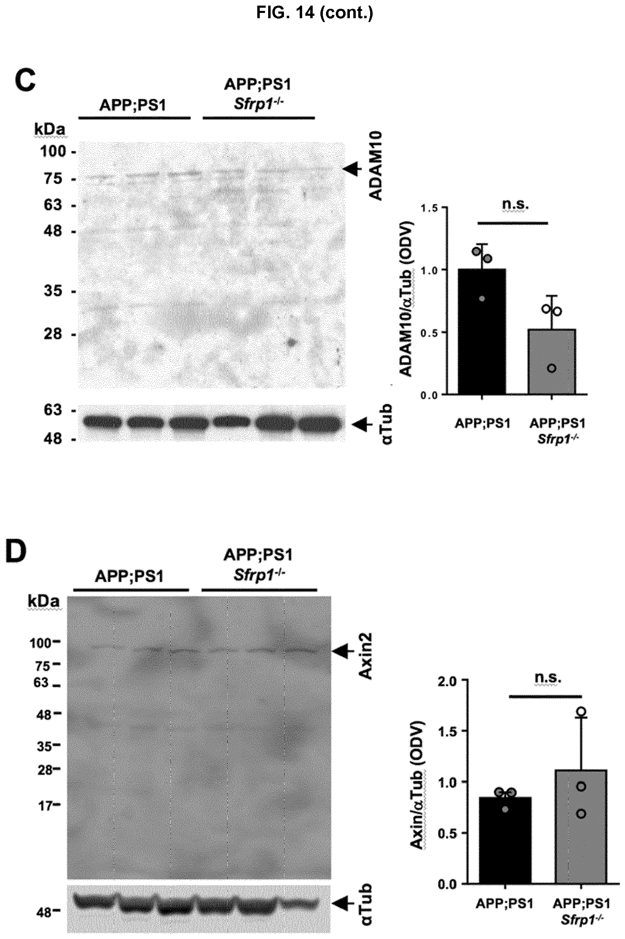

**Fig. 14****. The mRNA and protein levels of *APP, ADAM10, BACE1* and *Axin2* are not affected by Sfrp1 inactivation in APP;PS1 mice. A) RT-**PCR analysis of *App, Adam10, Axin2* and *GAPDH* mRNA from APP;PS1 and APP;PS1;*Sfrp1*^{-/-} brains. Note that mRNA levels are similar in both mouse lines, as normalized with control *GAPDH* levels. **B-D)** Western blot analysis of BACE1 (B), ADAM10 (C) and Axin2 (D) protein levels in APP;PS1 and APP;PS1;*Sfrp1*^{-/-} brains. Data were normalized to α-Tubulin levels used as a loading control and analyzed with Student-t test; n.s. not statistically significant.

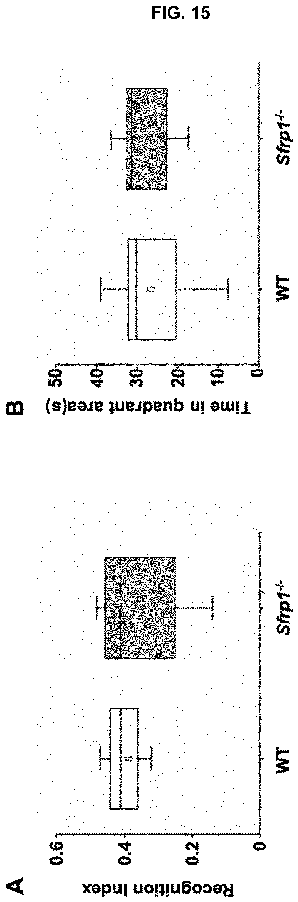

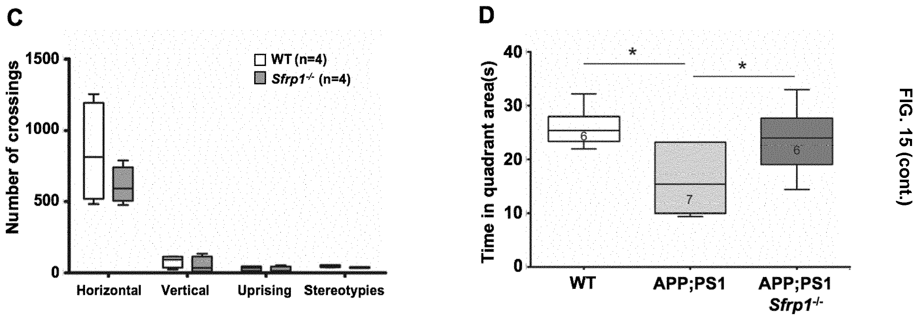

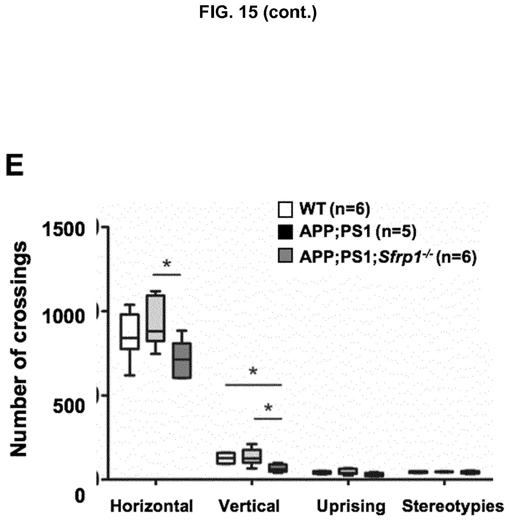

**Fig. 15****. Behavioral analysis of wt, *Sfrp1*^{-/-}, APP;PS1 and APP;PS1 *Sfrp1***^{**-**/-} **mice. A-C)** The graphs depict the recognition index **(A)**, spatial memory retention (**B**) and number of crossing (**C**) of wt and *Sfrp1*^{-/-} 8 months-old mice subjected to the object recognition test, water maze and open field exploration, respectively. The behavior of *Sfrp1*^{-/-} mice was undistinguishable from that of wt mice. The graphs depict the time in quadrant (**D**) and number of crossing (**E**) of wt, APP;PS1 and APP;PS1;*Sfrp1*^{-/-} of 8 months-old mice subjected to water maze and open field exploration, respectively. APP;PS1 mice showed a poor memory, whereas APP;PS1;*Sfrp1*^{-/-} mice were undistinguishable from wt. APP;PS1;*Sfrp1*^{-/-} mice showed a slightly lower locomotor performance. Data are means ± s.e.m. The number (n) of analyzed animals is indicated in the bars. Statistical analysis was performed using Student t test (**A-C**) or One-way ANOVA followed by Tukey method (**D-E**)**;** **p*≤0.05.

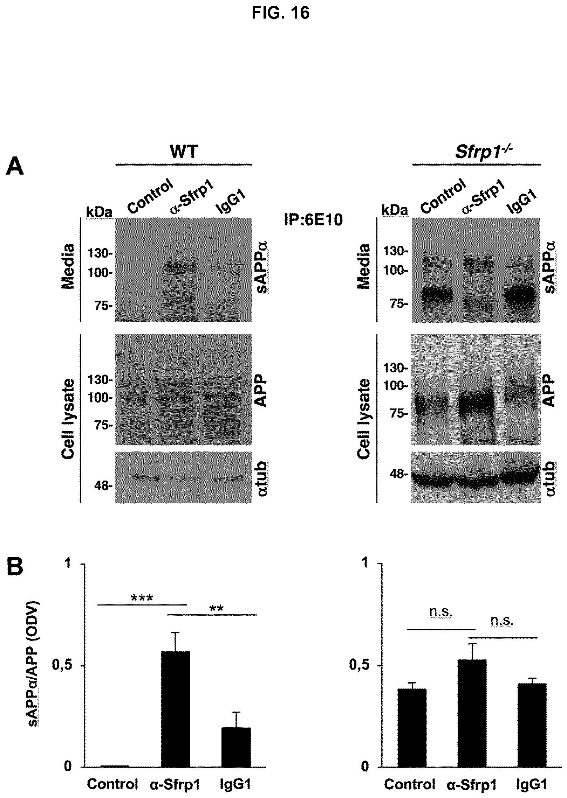

**Fig. 16****. mAb 10.5.6 neutralizes SFRP1 activity.** Cultured cells release in the culture medium the sAPPα peptide derived from ADAM10-mediated proteolytic processing of APP16. Sfrp1 binds to ADAM10 and prevents APP processing thus decreasing the amount of sAPPα in the media of cultured cells. **A)** Telencephalic neuroepithelial cells express high level of Sfrp1 mRNA therefore cultured telencephalic cells from E13.5 wt embryos release undetectable levels sAPPα in the medium, whereas those from *Sfrp1*^{-/-} release detectable amounts sAPPa. The addition of the 10.5.6 IgG1 anti-Sfrp1, but not that of an unspecific IgG of the same isotype, to the medium of wt cultures enhanced sAPPα release, indicating its ability of neutralizing Sfrp1 function. No changes in the amount of sAPPα were instead observed in the *Sfrp1*^{-/-} cultures. **(A)** The panels show Western blot analysis of media from wt and *Sfrp1*^{-/-} cultures immunoprecipitated with anti-Aβ (6E10) antibody and analyzed with the same antibody. sAPPα fragment is barely detectable in media from wt cultures but its levels increase at levels comparable to those observed in *Sfrp1*^{-/-} cultures upon treatment with anti-Sfrp1 mAb, whereas an unspecific IgG1 has no effect **(B)**. No changes in the amount of sAPPα were instead observed in the *Sfrp1*^{-/-} cultures (B). The data represent a typical experiment, which was repeated three times with similar results. Statistical analysis of these experiments is represented in B. Data were analyzed with Student-t test, **p≤0.01; ***p≤0.001; n.s. non significant.

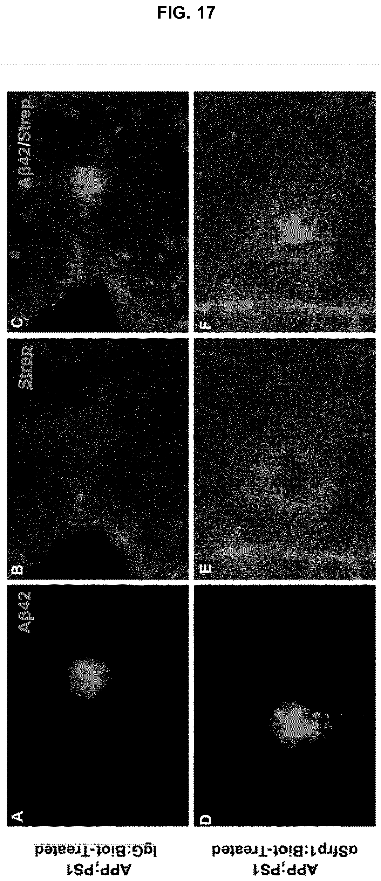

**Fig. 17****. Biotinylated anti-Sfrp1 antibodies enter the brain parenchyma via intravenous administration. A-F)** Frontal sections of the brain from APP;PS1 mice injected 24 hours before in the retro-orbital sinus with either biotinylated 10.5.6 mAb or unspecific mouse IgG1 as indicated in the panel. Sections were stained with streptavidin-POD followed by tyramide amplification and immunostained for Aβ42. There is accumulation of the 10.5.6 mAb in the brain parenchyma especially around APs and pia surface, whereas the unspecific IgG1 is poorly detected in comparable sections. Scale bar: 100 µm.

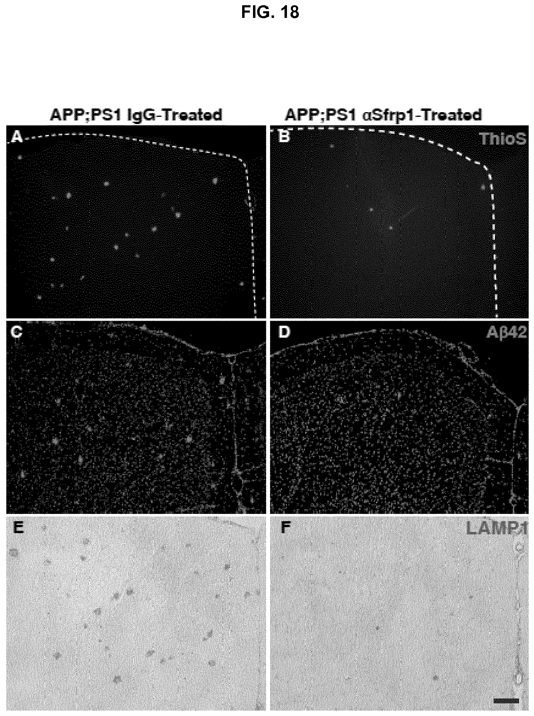

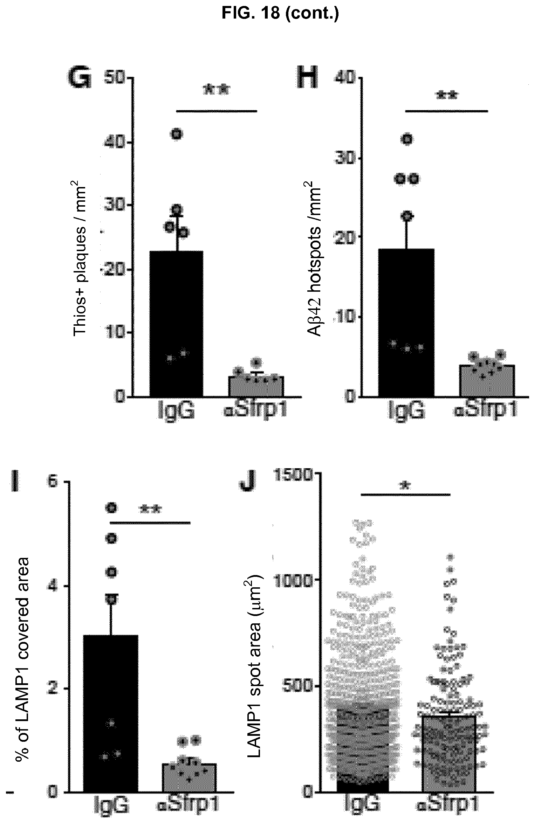

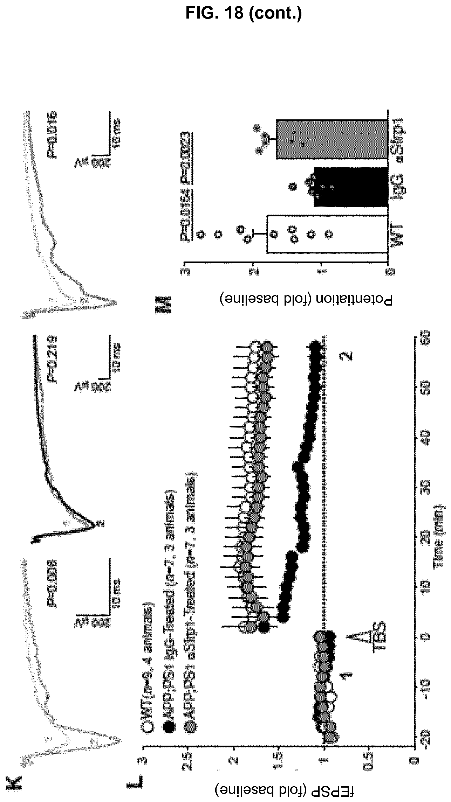

**Fig. 18****. Antibody-mediated neutralization of Sfrp1 activity counteracts the appearance of AD pathogenic traits in mice. (A-F)** Frontal cryostat sections of the cortex from 4-months-old APP;PS1 mice treated for 2 months with an unspecific mouse IgG1 or the anti-Sfrp1 IgG1 mAb 10.5.6, as indicated in the panels. Sections were stained with ThioS (**A,B**) or immunostained for Aβ42 (**D,E**) or LAMP1 (**E,F**). Scale bar: 100 µm. (**G-I**). Quantification of the number of ThioS+ and Aβ42+ APs and area covered by LAMP1 immuno-signal in α-IgG- and α-Sfrp1-treated APP;PS1 animals. (**J,K**) The distribution and percentage of LAMP1 hotspots of a given size is also illustrated. Sfrp1 neutralization prevents the appearance of larger and more deleterious LAMP1 hotspots. Data are means ±s.e.m. There is a significant decrease in APs and LAMP1 staining in mice treated with α-Sfrp1 mAb. The number (n) of quantified animals is indicated in the bars. Data were analyzed by Student's t-test, **p*<0.05; ***p*≤0.01; ****p*<0.001. **K-M)** Blockade of SFRP1 function rescues synaptic plasticity in APP;PS1 mice. **K)**. Representative traces for electrophysiological recordings with acute hippocampal slices from wt (left) or APP;PS1 mice treated with mouse IgG1 (middle) or the □□Sfrp1 mAb (right). Baseline responses (light traces) are taken between 10 and 0 minutes before LTP induction, and potentiated responses (dark traces) are taken between 50 and 60 minutes after LTP induction. L) Time course of CA3-to-CA1 synaptic transmission (fEPSP slope) during an LTP experiment with acute hippocampal slices from wt (white symbols) or APP;PS1 mice treated with mouse IgG1 (black symbols) or the α-Sfrp1 mAb (red symbols). LTP was induced by θ-burst stimulation (TBS) and recorded for 60 min post-induction, following at least 20 min of stable baseline. **M**) Quantification of average synaptic potentiation from the last 10 minutes of the time course shown in **L**. Data were analyzed with Mann-Whitney test.

### EXAMPLES

### Example 1. Elevated levels of Secreted-Frizzled-Related-Protein 1 contribute to Alzheimer's disease pathogenesis.

The present invention is focused on *Sfrp1*, a secreted, highly dispersible protein that binds to ADAM10 thereby acting as an endogenous negative regulator of its activity. It was hypothesized herein that upregulation of brain SFRP1 levels could be a common trait of AD patients.

The development of a specific and sensitive ELISA, based on newly generated α-SFRP1 mAbs (Fig. 1), showed a correlation between SFRP1 upregulation and AD pathological state. Indeed, SFRP1 protein was elevated in both the TBS and RIPA-soluble fractions of entorhinal and frontal cortex extracts from AD patients at pre-symptomatic, mild and advanced neuropathological stages of the disease (Braak and Braak (BB) BB I-II/0-A; III-IV/0-C; BB V-VI/B-C), when compared to age-matched control samples (Fig. 2A, C). The increase was particularly evident in both fractions of the entorhinal cortex at early and intermediate disease stages, but less so in the soluble fraction at late stages, when SFRP1 levels were instead the highest in the RIPA fraction (Fig. 2A). The SFRP1 increase observed in the soluble fraction correlated -positively and negatively, respectively- with the amount of Aβ and sAPPα present in the same extracts (Fig 2J, K), as expected if SFRP1 inhibits APP non-amyloidogenic processing.

Protein increase was paralleled by *SFRP1* transcriptional up-regulation especially at late disease stages -with no parallel significant changes in the expression of BACE1, APP and ADAM10 at least at early and intermediate stages (Fig. 3)-, as shown by mRNA quantification in a similar characterized cohort of AD patients and age-matched controls (Fig. 2B, D). Immunohistochemical analysis of human frontal cortex from AD patients revealed a strong and specific accumulation of SFRP1 in Aβ+ (Fig. 2E) and ThioS+ plaques (APs; Fig. 4B), and, less frequently, in aggregates with a poor Aβ+ (Fig. 2F arrow) or ThioS (Fig. 4C) signal. SFRP1 localization in APs and its presence in the RIPA-insoluble fraction raised the possibility of an interaction between SFRP1 and Aβ peptides. *In vitro* assays confirmed this possibility, showing that the two molecules form SDS-resistant complexes with molecular weights compatible with combinations of both SFRP1 and Aβ multimers (Fig. 2M, N). The binding of Aβ peptides to SFRP1 appeared stronger than that previously reported for BSA (Fig. 4H, I).

Besides its detection in APs, SFRP1 immunoreactivity was also consistently found in elastin+ blood vessels (Fig. 4D, E) colocalizing with Aβ deposits (Fig. 2G). SFRP1 was also present in some GFAP+ reactive astrocytes surrounding the APs (Fig. 2H) and in activated Iba1+ microglia infiltrated in the APs (Fig. 2I). Poor immuno-histochemical signal was instead detected in the cortex of control individuals (Fig. 4G).

Furthermore, Western blot (WB) analysis of choroid plexus samples from AD and control individuals revealed SFRP1 enrichment in the AD samples (Fig. 4J). Because the choroid plexus is the main source of CSF proteins with an unbalanced function AD patients, SFRP1 content in CSF samples of another characterized cohort AD patients and control individuals was measured, finding a significantly higher protein concentration in patients than in controls (Fig. 2L).

Together these data demonstrate a correlation between elevated SFRP1 content and the presence of AD.

To analyze whether SFRP1 is causally linked to AD progression, we turned to APP;PS1 transgenic mice (males were used throughout the study) that express human mutant alleles of APP and presenilin-1 (PS1, a subunit of γ-secretase complex) and that are commonly used as a model of amyloidosis. Similarly to what was observed in human tissue, a mouse-adapted and highly specific ELISA on RIPA detergent-soluble fraction of APP;PS1 brains showed an age-depended significant increase of *Sfrp1* levels, already evident in pre-symptomatic 2 months-old animals (Fig. 5A, B), in agreement with what was observed in the entorhinal cortex of BI-II stage patients (Fig. 2A). This increase was further confirmed by WB analysis of brain extracts from APP;PS1 brains (Fig. 6A). Co-immunostaining with α-Sfrp1 and glial specific markers in wt (Fig. 6B, C) and APP;PS1 (Fig. 5D, E) mice showed that *Sfrp1* localized to the wall of the lateral ventricle and GFAP+ astrocytes and Iba1+ microglial cells as well as to laminin+ blood vessels (Fig. 6E, F). As observed in human samples, a strong and very specific Sfrp1 + signal was also detected in ThioS+ and Aβ+ APs (Fig. 5E-I; Fig. 7A-D) and in the choroid plexus of APP;PS1 mice with an intensity that increased with age (Fig. 5J-L). Co-IP studies using cortical extracts of 6 months old APP;PS1 mice confirmed that *Sfrp1* and Aβ also interact *in vivo* (Fig. 7E). Double RNAScope ISH with probes for *Iba1* and *Sfrp1* mRNA and *Sfrp1* ISH coupled to anti-GFAP further showed that in APP;PS1 brains *Sfrp1* is produced in astrocytes, microglial and choroid plexus cells, at levels higher than in wt (Fig. 7). Notably, glial cells surrounding ThioS+ plaques appeared to express twice as much *Sfrp1* mRNA than those away from the plaques (Fig. 7E).

In APP;PS1 brains- but also in AD patient- APP expression and processing occurs mostly in neurons, in which ADAM10 is found at the synapse. Consistent with the idea that *Sfrp1* released by glial cells can influence neuronal ADAM10 activity, *Sfrp1* co-IP with transgenic human APP in the synaptosomal fraction from 6 months old APP;PS1 mice whereas binding was undetectable in the wt fraction (Fig. 5U,V).

Given that *Sfrp1* expression and distribution in APP;PS1 mice mimicked that observed in human AD samples, it was next tested if the forced increase of *Sfrp1* levels in the brain of young APP;PS1 mice would accelerate AP deposition. It was first used heterozygous APP;PS1 mice, because of their late and slow AP build-up. Mice were transduced with lentiviral preparations expressing either *GFP* (LV-GFP) or *Sfrp1* (LV-*Sfrp1*-IRES-*GFP*) at 2.5 months of age and analyzed 3 months later. At this final age, APs were barely detectable in the cortex of het-APP;PS1 mice (not shown) or of mice transduced with LV-GFP (Fig. 5M,O,S). In contrast, mice exposed to LV-*Sfrp1*-IRES-*GFP* presented a significant build-up of Aβ+ APs, surrounded by CD45+ activated microglial cells (Fig. 5N,P,S) as commonly observed around APs. There was also an increase of significantly larger dystrophic neurites positive for LAMP1 (Fig. 5Q,R,T), a lysosomal glycoprotein that accumulates in degenerating neuronal processes. A similar effect was observed when 2 months old homozygous APP;PS1 mice were transduced with either one of the two LV preparations and analyzed 1 month after. In LV*-Sfrp1*-IRES-*GFP* transduced animals, Aβ+ APs, reactive gliosis and pTau+ dystrophic neurites outnumbered the few found in LV-GFP transduced (Fig. 8) or non-transduced (not shown) mice.

Together these results support the contention that elevated *Sfrp1* levels contribute to AP formation.

To further test the link between *Sfrp1* and amyloid deposition, it was performed the converse experiment using APP;PS1;*Sfrp1*^{-/-} mice, generated by crossing the APP;PS1 line with *Sfrp1*^{-/-} mice, which are viable, fertile and show no sign of neurodegeneration. Taking advantage of the *lacZ* reporter present in the *Sfrp1* null allele, it was first determined that both GFAP+ astrocytes and Iba1+ microglial cells, but not Neu+ neurons, are indeed the source of brain *Sfrp1* (Fig. 9). Next, the cortex of 4.5, 9 and 20 months-old APP;PS1 and APP;PS1;*Sfrp1*^{-/-} mice was immunostained with an anti-Aβ42 antibody (H31L21) that recognizes only the longer and more pathogenic Aβ forms present in APs and the surrounding protofibrillar accumulations. APP;PS1;*Sfrp1*^{-/-} cortex developed significantly less, smaller and more compact Aβ42+ (Fig. 10A-F, G; Fig. 11M) or ThioS+ (Fig. 11A-G) APs than their APP;PS1 counterparts. Similar results were found in brains of a different group of APP;PS1 and APP;PS1;*Sfrp1*^{-/-} mice probed with anti-Aβ₄₀₋₄₂ antibodies (Fig. 11H-L). In all cases, AP reduction correlated with a comparably significant decrease in LAMP1, Iba1, CD45 and GFAP immunoreactivity more evident in 4.5 months-old animals (Fig. 10J-U; Fig. 12).

Consistent with the observation that in the absence of *Sfrp1* APs are reduced, the amount of Aβ42 present in RIPA detergent-soluble fractions (Fig. 10H) and the levels of other β-secretase-derived APP fragments, i.e. CTFβ, present in 6 months-old APP;PS1;*Sfrp1*^{-/-} brains were significantly reduced when compared to those of age matched APP;PS1 brains (Fig. 10V,W; Fig. 13A-C), whereas those of sAPPα were significantly increased (Fig. 10I). Similarly, the proteolysis of Cdh2, another ADAM10 substrate, implicated in synapse stabilization, was also significantly enhanced in APP;PS1;*Sfrp1*^{-/-} when compared to APP;PS1 (Fig. 12C). These changes were not related to differences in brain *App* and *Adam10* expression, as the respective mRNA levels were similar in the two genotypes (Fig. 14A). No changes in BACE1 and ADAM10 protein expression were also detected (Fig. 14B, C).

Together these data indicate that, in the absence of *Sfrp1,* APP processing is shifted towards the non-amyloidogenic pathway, decreasing the accumulation of APP toxic forms, in agreement with the idea that α- and β- secretases compete for APP processing. *Sfrp1* co-immunoprecipitates with ADAM10 in cortical tissue and interferes with the proteolytical processing of different ADAM10 substrates. In further support of the interpretation that *Sfrp1* effects are linked to ADAM10, the shift in APP processing appeared independent from the well-established additional function of *Sfrp1* as Wnt signaling modulator. Indeed, brain mRNA and protein levels of *Axin2*, a read-out of Wnt/βcatenin signaling activation, was comparable in both genotypes (Fig. 12A, D).

To determine if *Sfrp1* inactivation protects APP;PS1 mice also from the appearance of behavioral deficits, recognition and spatial learning and memory in 8-months-old wt, *Sfrp1*^{-/-}*,* APP;PS1 and APP;PS1;*Sfrp1*^{-/-} mice, were compared using the novel object recognition (ORT) and Morris water maze tests. The behavior of *Sfrp1*^{-/-} mice was undistinguishable from that of wt mice, with proficient ORT discrimination indexes and with comparable spatial and locomotor skills (Fig. 15A-C). In notable contrast with the evident cognitive decline observed in APP;PS1, APP;PS1;*Sfrp1*^{-/-} mice showed a behavior similar to that of age-matched wt mice in both tests, despite a slightly lower locomotor performance (Fig. 10X,Y; Fig. 15D,E).

Together these data indicate that knocking *Sfrp1* out largely prevents the appearance of pathogenic characteristics in APP;PS1 mice, strongly delaying their progressive dysfunctions.

### Example 2. Generation of anti-SFRP1 monoclonal antibodies (mAbs).

mAbs against SFRP1 were generated as follows:
**Immunizations.** Four *Sfrp1*^{-/-} mice (129xBI6) of 12 weeks of age were immunized by intraperitoneal injection with 50 µg of human recombinant SFRP1 (Sigma-Aldrich MO, USA) emulsified with Complete Freund's adjuvant (FA, Sigma-Aldrich MO, USA). Animals were boosted twice (two weeks apart) with 25 µg of SFRP-1 emulsified with Incomplete FA. Effective immunization was determined by ELISA assay (see below) using a blood sample collected one week after the last immunization. The animals selected for hybridoma production were boosted again as above four days before cell fusion.
**Hybridoma cell production.** Fusion partner mouse myeloma Sp2/0-Ag14 cells were cultured and propagated in RPMI-1640 culture medium (Lonza Verviers, Belgium) supplemented with 10% Fetal Bovine Serum (FBS) (Hyclone, Thermo Fisher, Waltham, MA, USA). Splenocytes from the immunized donor mouse were mixed with the Sp2/0-Ag14 cells at a ratio of 3:1. Cells were washed twice with pre-warmed RPMI-164 and incubated with 50% Polyethylene glycol 1500 (Sigma-Aldrich MO, USA). Hybridoma cells were selected with Conacell-HY medium E supplemented with 0.4µM aminopterin (StemCell, Grenoble, France). The presence of antibody reactivity in culture supernatant was determined with capture ELISA and further confirmed with indirect ELISA, western blotting, and immunohistochemistry. Hybridomas from positive wells were cloned twice by limiting dilution in Conacell-HY medium E.
**Capture ELISA.** Mouse serum titration and screening of hybridoma supernatants were performed using the following capture ELISA. 96-well microtiter plates (Nunc Roskilde Denmark) were coated (2 h at 37°C, or overnight at 4°C) with 50 µl (3 µg/ml in 0.01 M phosphate buffered saline, PBS) of goat anti-mouse IgG (SouthernBiotech AL USA). Plates were washed 3 times with PBS containing 0,05%Tween 20 (PBST) and treated (37°C, 30 min) with 100 µl of 2% Bovine serum albumin (BSA) (Sigma-Aldrich MO, USA). Plates were washed again with PBST, and incubated (2 hr, 37°C) with 50 µl/well of mouse antisera (two-fold serial dilutions in PBST/BSA, starting from 1:10) or with hybridoma supernatants. Wells were washed with PBST and incubated (1 hr, RT) with 50 µl of biotin-labeled SFRP1 (0.4 µg/ml). Plates were further washed with PBST and incubated (30 min, 20°C) with 50 µl of streptavidin-HRP (1/2000). After extensive washing with PBST, the enzymatic reaction was developed in the dark for about 5 min using 100 µl/well of O-phenylenediamine (OPD; 0.5 mg/ml; Sigma-Aldrich, Missouri, USA) and 1/1.000v/v of H₂O₂ (33 %). The reaction was terminated by the addition of 50 µl of 3N H₂SO₄ and the resulting product measured at 492 nm in a microtiter plate ELISA reader.
**Isotype determination.** mAb isotype was determined with an ELISA (see below) using the following coating. ELISA wells were coated with 50 µl of goat anti-mouse IgG (3µg/ml PBS) for 2 h at 37°C followed by 1hr incubation with Hybridoma supernatants (50µl) and HRP conjugated anti-mouse IgG1, IgG2a, IgG2b or IgG3 (1/2000 dilution).
**Indirect ELISA.** ELISA plate wells were incubated for 2 h at 37°C with 50 µl of SFRP1 (1 µg/ml PBS). After washing 3 x with PBST, plates were blocked with 100 ul of 2% BSA for 30 min at 37°C, washed 3x times and incubated with 50 µl of well culture supernatants for 1 h at 37°C. After washing with PBST, wells were incubated with 50 µl of goat anti-mouse IgG-HRP (1:2000) for 30 min at 20°C. After washing, the reaction was developed and determined as described for the capture ELISA.
**Western blots.** E13.5 telencephalic tissues from wt and Sfrp1^{-/-} embryos were isolated and homogenized in lysis buffer (150mM NaCl, 1% NP40, 50mM Tris pH 8) containing proteinase inhibitors. Human recombinant SFRP1 protein (RD), tears (5µl) as positive controls, and telencephalic lysates were resolved in a 12% SDS-PAGE transferred to PDVF membranes, which were incubated with hybridoma supernatants. Signal was detected with ECL Select.
**Immunohistochemistry.** E12.5 wt and Sfrp1^{-/-} embryos were fixed by immersion in 4% paraformaldehyde/phosphate buffer for 4h. Tissue was then washed in PBS, incubated in 30% sucrose/PBS solution, embedded and frozen in a 7.5% gelatin/15% sucrose solution. Cryostat sections were blocked in PBS containing 0.2%Triton X100, 5%FCS and 1%BSA for 1h at RT. Sections were then isolated with clonal rings and incubated with hybridoma supernatants diluted (1:1) in the same buffer overnight at 4°C. After washing, sections were incubated with anti-mouse biotin conjugated antibodies for 1 h followed by Streptavidin-Alexa594.
**Characterization of the two mAbs designed in this invention.** mAb 10.5.6 was characterized as an IgG1 using the isotype determination analysis described above and further characterized by sequencing (see the sequences SEQ ID NO: 1 and SEQ ID NO: 2). mAb 17.08.13 was characterized as an IgG2b using the isotype determination analysis described above and further characterized by sequencing (see the sequence SEQ ID NO: 3).

### Example 3. In vivo α-Sfrp1 antibody treatment prevented the formation and enlargement of toxic amyloid plaques.

Among the generated mAbs, clone 10.5.6 secreted an IgG1 with SFRP1 neutralizing activity (Fig. 16). Biotinylation of the 10.5.6 mAb showed the mAb successfully reached the brain parenchyma of APP;PS1 mice 24 hrs after systemic injection through the retro-orbital sinus, accumulating around Aβ+ plaques (Fig. 17). Treating for two months, via the same route, 2-months-old APP;PS1 mice with a weekly administration of 10.5.6 mAb (100µg/injection) significantly reduced their cortical AP burden when compared to animals treated with a control unspecific mouse IgG1 (Fig. 18A-F). This change was associated to a comparable significant reduction in the number and size of LAMP1+ accumulation around the APs (Fig. 18G-I), indicating that α-Sfrp1 treatment prevented the formation and enlargement of toxic plaques (Fig. 18M), thereby limiting the area of dystrophic neurites that contribute to brain degeneration.

In a second set of experiments animals were treated with the same protocol but for three additional months and then it was tested whether *in vivo* blocking of SFRP1 would rescue synaptic plasticity deficits already documented for the APP;PS1 mice used in this study. Synaptic function was assessed by electrophysiological recordings of field excitatory postsynaptic potential (fEPSPs) between hippocampal CA3 and CA1 cells in acute slices prepared from wt or APP;PS1 treated mice. APP;PS1 animals injected with the control IgG showed a strong impairment in long-term potentiation (LTP), as compared to wt (Fig. 18N-P). This impairment was completely rescued when APP;PS1 were treated with SFRP1 neutralizing 10.5.6 antibody (Fig. 18N-P). Analysis of sAPPα and soluble Aβ peptides levels present in cortical extracts derived from the same set of α-Sfrp1 vs. IgG treated animals reveled a tendency to shifting APP processing towards the non-amyloidogenic pathways (Fig. 18J-L).

All in all, these data suggest a model in which SFRP1, produced by activated microglia and reactive astrocytes, diffuses into the parenchyma to inhibit ADAM10-mediated APP processing in neurons, thus enhancing pro-amyloidogenic APP processing and the production of toxic Aβ products that contribute to sustain AD progression. Manipulations of SFRP1 levels in APP;PS1 mice support this possibility and show that the absence or neutralization of SFRP1 activity suffices to prevent cognitive loss and synaptic function in this amyloidosis model. The accumulation of SFRP1 in APs and its significant upregulation in brain parenchyma and CSF of AD patients make also a strong case in favor of SFRP1 implication in disease pathogenesis.

The results recently obtained by the administration of an α-Aβ mAb (Aducanumab), which targets Aβ aggregated forms, support that decreasing AP burden and soluble oligomeric Aβ delays the disease. In mice, SFRP1 neutralization results in a decrease of pathogenic signs, proving that lowering the SFRP1 levels has a positive effect. Thus, targeting SFRP1, alone or in combination with Aducanumab, represents an AD therapeutic avenue.

### Example 4. Material and methods.

**Selection of cases and general human tissue samples processing.** Brain tissue was obtained from the Institute of Neuropathology HUB-ICO-IDIBELL and Clinic Hospital-IDIBAPS Biobanks and the CIEN Tissue Bank, CIEN Foundation, Instituto de Salud Carlos III. The post-mortem interval between death and tissue processing was between 3 and 16 hours. One hemisphere was immediately sectioned in the coronal plane at 1cm of thickness and selected areas of the encephalon were rapidly dissected, frozen on metal plates over dry ice, placed in individual air-tight plastic bags, numbered with water-resistant ink, and stored at -80°C until use. The other hemisphere was fixed by immersion in 4% buffered formalin for 3 weeks for morphological studies. Tissue samples were also fixed in 4% paraformaldehyde for 24 h and cryoprotected with 30% sucrose for 48h, frozen in liquid nitrogen and maintained at -80°C until use. Neuropathological study in all cases was routinely performed on 20 de-waxed paraffin sections comprising different regions of the cerebral cortex, diencephalon, thalamus, brainstem and cerebellum, which were stained with eosin and haematoxylin, Klüver-Barrera, and immunostained for microglia, GFAP, Aβ, p-Tau (clone AT8), α-synuclein, TDP-43, ubiquitin and p62. Neuropathological diagnosis of AD was based on BB classification adapted for paraffin sections. Cases with combined pathologies (i.e. Parkinson's disease, Tauopathy, vascular diseases or metabolic syndrome) were excluded from the present study. Age-matched control cases had not suffered from neurologic, psychiatric diseases or metabolic diseases (including metabolic syndrome) and had no abnormalities in neuropathological examination (except BB I-II). SFRP1, BACE1, APP and ADAM10 mRNA expression was determined using the entorhinal and frontal cortex. Only samples with acceptable RNA integrity number (RIN) values were used. SFRP1, Aβ, sAPPα protein/peptide levels were determined using the entorhinal and frontal cortex of AD patients at different Braak stages of the diseases and compared with age-matched controls. Samples were collected as described above. Human biological samples correspond to post-mortem brain donations to the CIEN Tissue Bank. CSFs were collected at the Dept. of Neurology outpatient unit for neurodegenerative disease (KBFZ) of the University of Bonn by MTH. CSF was obtained by lumbar puncture at position L3, centrifuged and aliquoted for further analysis. Turbid or blood contaminated samples were excluded from analysis.

**RNA purification.** RNA was purified with RNeasy Lipid Tissue Mini Kit (Qiagen, Hilden, DE) following the manufacturer's protocol. Samples' concentration was determined at A260 using a Nanodrop 2000 spectrophotometer (Thermo Fisher, Waltham, Massachusetts, USA). RNA integrity was determined using the Agilent 2100 BioAnalyzer (Agilent, Santa Clara, CA, USA).

**Retrotranscription reaction.** Samples with RIN values from 6.4 to 9.1 were retro-transcribed in the presence or the absence (to assess genomic DNA contamination) of MultiScribe Reverse Transcriptase using the High capacity cDNA Archive kit (Applied Biosystems, Foster City, CA, USA) following the manufacturer's guidelines and using Gene Amp® 9700 PCR System thermocycler (Applied Biosystems, Foster City, CA, USA).

**TaqMan Real Time PCR.** TaqMan PCR assays were performed in duplicate in 384-well optical plates using an ABI Prism 7900 Sequence Detection system (Applied Biosystems, Foster City, CA, USA). The TaqMan reaction mixture (10 µl) containing 4.5 µl cDNA was mixed with 0.5 µl 20x TaqMan Gene Expression Assays and 5 µl of 2x TaqMan Universal polymerase chain reaction (PCR) Master Mix (Applied Biosystems, Foster City, CA, USA). HPRT (hypoxanthine phosphoribosyltransferase) and AARS (alanyl-tRNA synthetase) probes were used in parallel assays performed in each sample and used as housekeeping controls. The reactions were performed using the following parameters: 50°C for 2 min, 95°C for 10 min, 40 cycles of 95°C for 15 s and 60°C for 1 min. All TaqMan PCR data were captured using the Sequence Detector Software (SDS version 1.9, Applied Biosystems, Foster City, CA, USA). Samples were analyzed with the double delta CT (ΔΔCT) method. Delta CT (ΔCT) values represent normalized target gene levels with respect to internal housekeeping controls (HPRT1 and AARS), selected for the replicating efficiency in human post-mortem brain tissue. ΔΔCT values were calculated as the ΔCT of each test sample minus the mean ΔCT of the calibrator samples for each target gene. The fold change was calculated using the equation 2^(-ΔΔCT). Results were analyzed with Kruskal-Wallis followed by post hoc Mann-Whitney test. Differences between groups were considered statistically significant at p-value: * p< 0.05.

**Mice.** Double chimeric transgenic APP;PS1 mice, expressing the human mutated APP (APP695swe) and presinilin1 (PS1-dE9) genes under the prion promoter that directs expression to the Central Nervous System, were crossed with *Sfrp1*^{+/-} mice to obtain APP;PS1;Sfrp1^{-/-} and APP;PS1;Sfrp1^{+/+} mice. Initial breeding pairs of the APP;PS1 mice were kindly provided by Dr. Torres-Aleman, Instituto Cajal, CSIC, Madrid. Homozygous male mice of age comprised between one and twenty months of age were used throughout the study, unless otherwise stated, with no inclusion/exclusion criteria other than genotype, sex and age. Within these criteria animals were randomly chosen among the available ones. The number of animals used for each experiment is indicated in the figures or their descriptions. The minimum number of animals required in each experiment was predicted using the G3*Power 3.1 program.

**Antibodies.** The following monoclonal antibodies were used: mouse anti-APP (clone 22C11, N-terminal, 1:1000, Chemicon, cat n° MAB348, batch LV1634989), mouse anti-Aβ (clone 4G8, that recognizes amino acids 17-24 of the human and mouse Aβ (1:500, Covance, cat n° SIG-39220, batch D11DF00836), mouse monoclonal anti-Aβ (clone 6E10, that recognizes amino acids 1-16 of the human Aβ and sAPPα (1:500, Covance, cat n° SIG-39320)), rabbit anti-Aβ (H31L21, that recognizes amino acids 36-42 of the human and mouse Aβ (1:1000, Thermo Fisher, cat n° 700254, batch RH240023)), mouse anti-sAPPα (clone 2B3, which recognizes sAPPα, 1:500, IBL, cat n° 11088, batch 11-227), rabbit anti-SFRP1 (1:2000, Abcam, cat n° ab126613), rat anti-CD45 (clone IBL-3/16, 1: 500, AbDSerotec, cat n° MCA1388), mouse anti-LAMP1 (clone ID4B, 1:200, DSHB), mouse anti-α-tubulin (clone B-5-1-2,1:5000, SIGMA), mouse anti-NeuN (clone A60, 1:500, Millipore, cat n° MAB377, batch LV1457494); rabbit anti-Axin2 (1:1000, Abcam #ab109307; lot GR1783799020); mouse anti-Cdh2 (Clone 3B9 1:500 Invitrogen #180224; lot 1553634A). Rabbit polyclonal antibodies were the following: anti-Aβ 1-40/42 (1:500, Millipore, cat n° AB5076, batch 2584867), anti-APP C-terminal (1:500, SIGMA, cat n° A8717, batch 045M4785V), anti-Sfrp1 (1:500, Abcam, cat n° ab4193), anti-GFAP (1:3000, DAKO, cat n° Z 0334, batch 20028619), anti-lba1 (1:2000; Wako, cat n° 019-19741, batch LKG5732); anti-BACE1 (1:1000 Calbiochem #195111 batch D0903996). Chick anti-βGal (1:500, Abcam, cat n° ab9361, batch 365032). Secondary antibodies were the followings: Alexa-488, Alexa-594- conjugated affinity-purified secondary anti-rabbit or mouse antibodies (1:2000 Molecular Probes) or biotin-conjugated anti-rabbit and anti-mouse IgG (1:500; Jackson ImmunoResearch, Cambridgeshire,UK) and visualized with peroxidase conjugated streptavidin (1:500, Jackson ImmunoResearch).

**Semi-quantitative PCRs.** RNA from mouse cortical hemispheres was purified using PureLink RNA Mini Kit (Ambion) following manufacturer's protocols. Samples concentration was determined at A260 using a Nanodrop 2000 spectrophotometer. RNA was retro-transcribed using iScript cDNA Synthesis Kit (BIO-RAD). cDNA concentration was determined at A230 using the same Nanodrop. Reactions to amplify fragments of the mouse Axin2, APP, Adam10 and glyceraldehy-3-phosphate dehydrogenase (GAPDH) were performed with the following primers' pairs: GAPDH: fw, 5'-accacagtccatgccatcac-3' (SEQ ID NO: 7), rev, 5'-tccaccaccctgttgctgta-3' (SEQ ID NO: 8), (452 bp amplicon); Axin2 fw, 5'-ctccttggaggcaagagcttc-3' (SEQ ID NO: 9), rev, 5'-gactggccacgcagcaccgct-3' (SEQ ID NO: 10) (675 bp amplicon); APP fw: 5'-atgtgcagaatggaaagt-3' (SEQ ID NO: 11), rev: 5'-cagcatacaaactctacc-3' (SEQ ID NO: 12), (424 bp amplicon) and Adam10 fw, 5'-gctgtgattgctcagatatccag-3' (SEQ ID NO: 13), rev, 5'-tctttgcactggtcactgta-3' (SEQ ID NO: 14), (720 bp amplicon).

**In situ hybridization (ISH) and immunohistochemistry (IHC).** Mice were anesthetized with pentobarbital or ketamine/xilacine and transcardiacally perfused with 4% paraformaldehyde (PFA) in phosphate buffer (PB). Brains were extracted and post-fixed for 24h at 4°C. Tissue was then washed in PB saline (PBS), incubated in a 30% sucrose/PBS solution, embedded and frozen in a 7.5% gelatin, 15% sucrose solution. ISH was performed with RNAscope 2.5 Duplex detection technology (ACDbio) in 10-15 µm cryostat sections, using a probe for mouse Sfrp1 mRNA (channel C1, detected in red) and a probe for mouse Iba1 mRNA (channel C2, detected in blue). ISH for Sfrp1 was combined with IHC for GFAP as described below. IHC was performed on free-floating 50 µm vibratome or 6 µm paraffin sections from frontal human cortices and on 10-15 µm mouse brain cryostat sections. In the case of Aβ and Sfrp1 co-immunostaining, sections were treated in 90% formic acid for 10 minutes or boiled in citrate buffer (10mM, pH:6) and then with 0.3% H₂O₂ in methanol for 20 min. After several washes in PBS, sections were treated with M.O.M. blocking reagent (Vector Labs), incubated in PBS containing 0.5% Triton X100, 5% FCS and 1% BSA for 2h at RT, and thereafter with anti-Sfrp1 10.5.6 mAb (1:2000) or anti-Sfrp1 rabbit polyclonal (Abcam) and anti-Aβ_{1-40/42} (4G8 or rabbit polyclonal, 1:500) antibodies diluted in the same buffer overnight at 4°C. After washing, sections were incubated with secondary antibodies for 2h. AP in human and mouse tissues were visualized with 0.01% ThioflavinS (SIGMA) in 50% EtOH and differentiated in 50% EtOH. In the case of Sfrp1 and GFAP or Iba1 and Aβ42 and CD45 or LAMP1 co-immunostaining, sections were pre-treated as above and incubated with appropriate antibodies overnight at 4°C. Tissue was analyzed with fluorescent conventional or confocal microscopy.

**Synaptosome isolation.** Synaptosomes were isolated from wt and APP;PS1 hemispheres. The synaptosome enriched fractions were solubilized in 147 mM NaCl, 3 mM KCI, 10 mM Glucose, 2 mM MgSO₄, 2mM CaCl₂, 20 mM Hepes, 0.1% SDS, 0.1% Na-Deoxicolate, 1% Triton X-100 plus protease inhibitors (Roche).

**Immunoprecipitation and Western Blot analysis.** To detect APP and the derived proteolytic peptides, cortical hemispheres from wt, APP;PS1;Sfrp1^{-/-} and APP;PS1 mice were homogenized in Tris-NaCl soluble buffer and centrifuged. The resulting pellets were homogenized in RIPA lysis buffer and processed for immunoprecipitation. After immunoprecipitation with anti-Aβ 6E10 mAb, beads were re-suspended in NuPAGE LDS sample buffer (4X) (Thermo Fisher, Waltham, MA, USA) without 2-mercaptoethanol, heated at 97°C on a shaker for 10 min, centrifuged and the supernatants were resolved in 10-20% Tris-Tricine gels (BIO-RAD). Gels were transferred to nitrocellulose membranes by dry iBlot (Invitrogen). The membranes were then boiled in PBS for 7 min, stained with Ponceau red solution, washed in TBST, and incubated sequentially in PBS containing 0.05% Tween, 10% nonfat milk and 0.1% BSA and then with a6E10 mAb solution, followed by peroxidase-conjugated secondary antibody. Signal was visualized with the ECL Advanced Western Blotting Detection Kit (Amersham). Membranes were stripped with Re-Blot Plus Strong Solution (Millipore) and re-blotted with the 4G8 mAb or anti-APP-C-terminal polyclonal antibody. To detect SFRP1-Aβ interaction, co-immunoprecipitation studies were performed using RIPA brain extracts from APP;PS1 and APP;PS1;Sfrpl^{-/-}. Co-IP were performed with either anti-Aβ (6E10), anti-Sfrp1 or IgG isotype control antibodies, followed by Western Blot with either anti-Sfrp1 or anti-Aβ antibodies, respectively. Co-IP studies to determine the possible interaction of SFRP1 to APP fragments in the synaptosome-enriched fraction from wt and APP;PS1 was performed following the same procedure.

**Protein-protein binding assays**. Nanomolar concentrations of recombinant human SFRP1 (RD cat: 5396-SF-025) and human Aβ1-42 peptides (Invitrogen, cat: 03-111, prepared to obtain minimal peptide aggregation) were mixed in protein Lobind tubes (Eppendorf) for 24 hrs at 37°C. NuPAGE LDS sample buffer (4X) was added to the tubes, boiled for 5 min and subjected to Western blot analysis. Microplate-based solid-phase protein-protein binding assays was performed by immobilizing in wells 200 ng/well of either human Aβ1-42 peptides, BSA, used as a positive control because previously described as an Aβ interactor; or heparin, a described SFRP1 binding partner. Immobilization was performed overnight at 4°C. The wells were then washed with TBS 3x and incubated with two different SFRP1 concentrations for 2 hrs at RT. In the converse experiment, BSA, heparin or SFRP1 were immobilized overnight at 4°C and two different concentrations of Aβ1-42 were added for 2 hrs at RT. The wells were washed 6X in TBS and 2X sample buffer containing β-mercaptoethanol was added to the well. The content was recovered by scrapping, boiled for 5min and analyzed by SDS-PAGE followed by WB using anti-Sfrp1 or anti-Aβ antibodies, respectively.

**Lentiviral production and injection.** Lentiviral preparations expressing GFP or SFRP1-IRES-GFP were obtained by transient transfection of mycoplasma-free HEK-293T cells, originally obtained from the ATCC. Cells were transfected employing a three plasmid HIV-derived and VSV pseudotyped lentiviral system kindly provided by M.K. Collins, University College London, UK; A. Thraser, Institute of Child Health, UK; and D. Trono, Ecole Polytechnique Fédérale de Lausanne, Switzerland. Culture supernatants were collected two and three days after transfection and ultra-centrifuged. The pellets containing the lentiviral particles were re-suspended in PBS (1x10⁸ TU/ml). Mice were anesthetized and placed in a stereotaxic frame. Small volumes (2.5 µl) of Sfrp1-GFP or GFP lentiviral particles were injected unilaterally in the frontal cortex using appropriate stereotaxic coordinates. Sham-operated animals served as additional controls. One or three months post-injection, mice were sacrificed and their brains were analyzed by IHC as described above. Six male mice were used for each experimental condition.

**ELISA assays**. In order to assess the levels of SFRP1 in human CSF samples, an ELISA assay was developed herein using the mAbs 10.5.6 (IgG1) and 17.8.13 (IgG2b) for capture and detection, respectively. 96-well microtiter plates (Nunc Roskilde Denmark) were coated overnight at 4°C with 50µl/well of goat anti-mouse IgG1 (SouthernBiotech AL USA) at 3µg/ml in PBS. Plates were washed 3 times with PBS containing 0.05%Tween 20 (PBST) and blocked (37°C, 30 min) with 100µl/well of 2% BSA in PBST (Sigma-Aldrich MO, USA). Plates were washed again with PBST and incubated (2 hrs, 37°C) with 50 µl/well of anti-Sfrp1 mAb 10.5.6 (1:10 dilution of hybridoma supernatant in blocking buffer). Wells were washed with PBST and incubated (2 hr, 37°C) with 50µl/well of samples diluted 1:10 and 1:20. After extensive washing with PBST, wells were incubated (1 hr, 37°C) with 50µl/well of anti-Sfrp1 17.8.13 (undiluted hybridoma supernatant). Plates were further washed with PBST and finally incubated (30 min, RT) with 50µl/well of goat anti-mouse IgG2b HRP (SouthernBiotech AL USA; 1:2000). After 3 washes with PBST, the enzymatic reaction was developed in the dark for about 5 min using 100 µl/well of O-phenylenediamine (OPD; 0.4 mg/ml diluted in Citrate-phosphate buffer pH5 0,1M; Sigma-Aldrich, Missouri, USA) and 1/1000 v/v of H₂O₂ (33%). The reaction was terminated by the addition of 50µl/well of 3N H₂SO₄ and the resulting product measured at 492 nm in a microtiter plate ELISA reader.

**Mouse and human cortical samples.** Human SFRP1 levels from cortex samples and mouse SFRP1 levels of brain lysates were determined in soluble (TBS) and RIPA fractions. The fractions were analyzed in a modified capture ELISA performed as follow. 96-well microtiter plates (Nunc Roskilde Denmark) were coated overnight at 4°C with 50µl/well of 1.5µg/ml purified anti-SFRP1 mAb 10.5.6 in PBS. Plates were washed 3X with PBST and incubated for 3hrs with 2% BSA in PBST at RT, washed with PBST, and incubated 2hrs at 37°C with 50µl/well of brain tissue samples, at protein concentration of 0.1µg/µl, previously quantified by BCA protein assay kit (Thermo Fisher, Waltham, MA, USA). Wells were washed with PBST and incubated for 1hr at 37°C with 50µl/well of biotin-labelled anti-SFRP1 mAb 17.8.13 (using the EZ-Link NHS-PEO4-Biotinylation Kit, Thermo Fisher Waltham, MA, USA, following the manufacturer's procedure) at 1µg/ml. Plates were further washed with PBST and incubated for 1h at RT with 50µl/well of Streptavidin-POD 1:2000 (Jackson Laboratory). After extensive washing with PBST, the enzymatic reaction was developed at RT in the dark for 20 min using 100µl/well of Tetra-methyl-benzidine liquid substrate (TMB slow kinetic form, Sigma Aldrich). The reaction was terminated by the addition of 100µl/well of HCI 2N and the resulting product was measured at 450nm in a microtiter plate ELISA reader FLUOstar OPTIMA (BMG LABTECH).

**ELISA assays for Aβ1-42 and sAPPα**. The levels of Aβ1-42 and sAPPa in human cortical samples and in the cortex from APP;PS1;Sfrp1^{-/-} and APP;PS1 mice treated with antibodies were determined in the soluble (TBS) fraction using the human Aβ42 ELISA Kit (Invitrogen, cat: KHB3441), the human-rat Aβ42 ELISA kit (WAKO, cat: 292-64501) and the human sAPPa ELISA kit (MyBioSource, San Diego, CA, USA, cat: MBS915453). Samples were analyzed blindly and in duplicates.

**Determination of SFRP1 neutralizing activity by mAb 10.5.6.** To determine the effect of possible neutralizing function among the mAbs generated as described above, it was tested the ability of the mAb to favor the generation of sAPPα in the culture medium of primary cultures of telencephalic neuroepithelium from E13.5 wt embryos. Parallel cultures from Sfrp1^{-/-} embryos were used as control. See the description of Fig. 12 for further explanations. Cultures were obtained as follow. Brains were removed in ice-cold Hank's solution (HBSS) and cortices were dissected, after removing the meninges. The samples were trypsinized, plated at a density of 5×10⁵ cells/cm² in dishes coated with poly-D-lysine (20 µg/ml; Sigma) and cultured in Neurobasal supplemented with B27 nutrients (Gibco BRL). After 24h, the medium was replaced with Neurobasal/B27 alone or containing hybridoma supernatant (1/1 volume) or unspecific muse IgG1 and cultured for additional 48 h. The media were collected and the cells were lysed, and all samples further processed for western blot analysis as described above.

**Intravenous administration of anti-SFRP1 antibody**. In a first set of experiments, mAb 10.5.6 anti-SFRP1 or an unspecific mouse IgG1 used as control were labelled with biotin using the EZ-Link NHS-PEO4-Biotinylation Kit (Thermo Fisher, cat: 21455). The antibodies were injected through the retro-orbital sinus and the animals were perfused after 24 hours and prepared for histological analysis as described above. The presence of the biotinylated mAb was determined by incubating the corresponding cryostat sections with streptavidin-POD (1:500) followed by incubation with tyramide (Cell Signaling). In a second set of experiments two months-old APP;PS1 mice were injected through the retro-orbital sinus once a week for two months, with 100µl of purified mAb 10.5.6 anti-SFRP1 or a unspecific IgG1 as a control (1µg/µl). After treatment, animals were perfused and prepared for histological analysis as described above. In a third set of experiments, 2 months old animals were injected once a week with 100µg of purified mAb 10.5.6 anti-SFRP1 or an unspecific IgG1 as a control for five months. After treatment, animals were prepared as described below for electrophysiology recordings and biochemical studies.

**Behavioral studies.** Behavioral studies were performed with 8-month-old male animals maintained in temperature and humidity-controlled conditions. Animals were subdivided according to the genotype, sex and age. The sample size used in each condition is indicated in the description of the figures. The investigator was blinded to the group allocation during the experiment. Locomotor activity was assessed with the Open field exploration test using an automated vertical and horizontal activity-monitored cage (25x25x25 cm). After 1h activity room habituation, animals were carefully introduced in the activity cage and their locomotor activity was automatically registered for 5 min. Recognition memory was determined with the novel object recognition test (ORT). In the habituation trial, animals were let explore a 36x56x30 cm field for 20 min. The day after, animals were first let explore the same arena containing in opposite corners two identical objects (A) for 10 min (acquisition). After 3h, mice were exposed to the familiar object (A) and a novel object (B) for 10 min (test). The total time dedicated to explore each one of the two objects was recorded. The recognition index was calculated as the ratio of the difference in time exploring the novel and familiar object and the total time spent exploring both objects, which made it possible to adjust for any differences in total exploration time. The Morris water maze test was used to assess spatial memory. Animals were placed in a pool of 100 cm in diameter and 40 cm in height, containing in one quadrant a clear 8x8 cm platform placed 1 cm below the surface of the water heated at 22°C ±1. Several visual cues surrounded the pool. During the habituation trial, mice were placed in the pool bearing no platform for 1 min and their performance and possible quadrant preference was scored. After 24 hr, animals were subjected to 4 trials/day with 30 min inter-trial-intervals for 4 days (16 trials total). Swim distance, speed and time spent to encounter the platform were recorded. Animals were guided to and maintained on the platform for 20s if unable to find it by themselves after 1 min. Scape latency was expressed as time needed to reach the platform. During the test trial, animals were placed in the center of pool with no platform for 60s. Time in the quadrant area was expressed as the amount of time spent in the quadrant containing the platform.

**Electrophysiology recordings.** Coronal acute hippocampal slices (300 µm thick) were prepared from mice after antibodies treatments described above. Mice were briefly anaesthetized using dry ice sublimated with water, and the brain removed and placed in partially frozen Ca2+-free dissection medium (10 mM D-Glucose, 4 mM KCI, 26 mM NaHCO₃, 234 mM sucrose, 5 mM MgCl₂, 1:1000 Phenol Red) saturated with 5% CO₂/95% O₂. Coronal slices were cut using a Leica VT1200S vibratome and let recover for 1 hr at 32°C in a submersion-type incubation chamber containing artificial cerebrospinal fluid (ACSF: 119 mM NaCl, 2.5 mM KCl, 2.5 mM CaCl₂, 1.2 mM MgCl₂, 26 mM NaHCO₃, 1 mM NaH₂PO₄, 11 mM glucose; pH 7.4). Slices were then transferred to the recording chamber, and maintained at 25 °C under perfusion with ACSF saturated with 5% CO₂/95% O₂. Electrically-evoked field excitatory postsynaptic potentials (fEPSPs) were recorded in the stratum radiatum of CA1. Bipolar stimulation electrodes were placed among Schaffer collaterals and glass recording electrodes (1-2 MΩ; filled with ACSF) placed ∼200 µm away in the direction of fiber projection. Stimulation frequency during baseline was 0.066 Hz. After acquiring a stable baseline transmission of at least 20 min, LTP was induced with a theta-burst protocol (4 pulses at 100 Hz, with the bursts repeated at 5 Hz and each tetanus including three 10-burst trains separated by 15 s).

**Quantification procedures and statistical analysis.** For each type of experiment, a pilot study was performed. It was then used the freely available statistical power analysis program G3*Power 3.1 to determine the minimal sample size needed in each set of experiment. The criteria of a p value of a maximum of 0.05 and a power of at least 0.8 was used. ISH or IHC preparations were visualized and captured, at the same exposure settings for each antibody, with a DM5000 microscope equipped with a DFC350Fx monochrome camera or a DFC500 color camera (Leica Microsystems). Captured areas from at least six sections separated by 200 µm were analyzed per each animal. The number of quantified animals is indicated in each plot and exemplifies a representative group among the total of analyzed animals (5-10 per group). Intensity of immune-positive signals, number of AP burden and area of LAMP1+ dystrophic neurites were quantified using ImageJ software (U.S. National Institutes of Health, Bethesda, Maryland, USA). Results were generally analyzed by Student's t-test but statistical tests used in each case are indicated in the corresponding figure description. Statistically significant differences were considered and represented by *p<0.05, **p<0.01, ***p <0.001. The experiments were analyzed by three blinded independent researchers. Behavioral tests were statistically analyzed as follow. The normality of the distribution was tested by Kolmogorov-Smirnov test and homoscedasticity was tested by Bartlett's test. Differences between groups were analyzed by ANOVA and two-sided test. Analysis was performed with the SPSS statistical package version 20.0, using a significance level of p ≤0.05.

## Claims

1. Use of the *Sfrp1* gene, the SFRP1 mRNA or the SFRP1 protein as a therapeutic target for designing and/or screening molecules, compounds, compositions or drugs useful for the treatment and/or prevention of Alzheimer's Disease.

2. A monoclonal antibody that specifically binds the Secreted Frizzled Related Protein 1 (SFRP1) protein, wherein the light chain of the variable region of said monoclonal antibody comprises the amino acid sequence of SEQ ID NO: 1 and the heavy chain of the variable region of said monoclonal antibody comprises the amino acid sequence of SEQ ID NO: 2.

3. The monoclonal antibody according to claim 2, wherein said antibody is conjugated with a detection system and/or is immobilized in a support.

4. A kit for the diagnosis of Alzheimer's Disease that comprises the monoclonal antibody according to any of claims 2 or 3.

5. The kit according to claim 4, that further comprises a second monoclonal antibody that specifically binds the SFRP1 protein, wherein the heavy chain of the variable region of said second monoclonal antibody comprises the amino acid sequence of SEQ ID NO: 3.

6. The kit according to claim 5, wherein the second monoclonal antibody is conjugated with a detection system and/or is immobilized in a support.

7. A pharmaceutical composition comprising an inhibitor of the SFRP1 protein or *Sfrp1* gene biological function.

8. The pharmaceutical composition according to claim 7, wherein the inhibitor is the monoclonal antibody according to any of claims 2 or 3.

9. The pharmaceutical composition according to claim 8, which further comprises a second monoclonal antibody that specifically binds the SFRP1 protein, wherein the heavy chain of the variable region of said second monoclonal antibody comprises the amino acid sequence of SEQ ID NO: 3.

10. An inhibitor of the SFRP1 protein or *Sfrp1* gene biological function for use as a medicament.

11. An inhibitor of the SFRP1 protein or *Sfrp1* gene biological function for use in the treatment and/or prevention of Alzheimer's Disease.

12. An inhibitor of the SFRP1 protein or *Sfrp1* gene biological function for use according to any of claims 10 or 11, wherein the inhibitor is the monoclonal antibody according to any of claims 2 or 3.

13. Use of the monoclonal antibody according to any of claims 2 or 3 or the kit according to any one of claims 4 to 6 in the *in vitro* diagnosis of Alzheimer's Disease.

14. Use according to claim 13, wherein the diagnosis of Alzheimer's Disease is performed by immunoassay, preferably by ELISA.

15. A method for the *in vitro* diagnosis of Alzheimer's Disease in a subject that comprises the following steps:
a. Contacting an isolated biological sample with the monoclonal antibody according to any one of claims 2 or 3 or with the kit according to any one of claims 4 to 6,
b. incubating the mixture of step (a) under conditions that allow the binding of the antibody to its antigen,
c. detecting and quantifying the amount of SFRP1 protein in the mixture of step (b),
d. comparing the amount obtained in step (c) to a standard value obtained from the quantification of the amount of SFRP1 protein in a biological sample isolated from a healthy subject, and
e. assigning the subject to the group of patients suffering from Alzheimer's Disease when the amount obtained in step (c) is significantly higher than the standard value.

16. The method according to claim 15, wherein steps (a) to (c) are performed by immunoassay, preferably by ELISA.

17. The method according to any of claims 15 or 16, wherein the isolated biological sample is a brain sample, preferably a brain cortex sample, or cerebrospinal fluid.
